# EUROPEAN PATENT SPECIFICATION

(11) **EP 2 361 081 B1**
(45) Date of publication and mention of the grant of the patent: **29.06.2016**
(21) Application number: 09820843.2
(22) Date of filing: 13.10.2009
(51) Int. Cl.: A61K 9/24, A61P 25/34

(54) **MULTI PORTION INTRA-ORAL DOSAGE FORM AND USE THEREOF**
AUS MEHREREN PORTIONEN BESTEHENDE INTRAORALE DOSIERFORM UND IHRE VERWENDUNG
FORME POSOLOGIQUE INTRA-ORALE À PARTIES MULTIPLES ET SON UTILISATION

(30) Priority: 14.10.2008 SE 0802189
(43) Date of publication of application: 31.08.2011
(73) Proprietor: McNeil AB, 25109 Helsingborg (SE)
(72) Inventor: LINDELL, Katarina, S-241 93 Eslöv (SE); THYRESSON, Kristina, S-226 57 Lund (SE); NICKLASSON, Fredrik, S-237 31 Bjärred (SE); BUNICK, Frank, Randolph New Jersey 07869 (US); LUBER, Joe, Quakertown Pennsylvania 18951 (US); HUGERTH, Andreas, S-237 33 Bjärred (SE)
(74) Representative: Kirsch, Susan Edith
(86) International application number: PCT/SE2009/051163
(87) International publication number: WO 2010/044736

(56) References cited:
- EP-A2- 1 107 730
- EP-A2- 1 190 721
- EP-A2- 1 190 721
- WO-A1-01/37814
- WO-A1-01/37814
- WO-A1-91/06288
- WO-A1-91/06288
- WO-A1-91/09599
- WO-A1-91/09599
- WO-A1-2005/121312
- WO-A2-00/13662
- US-A- 5 879 710
- US-A1- 2008 187 589
- DELVAUX M ET AL: "L'aide a l'arret du tabagisme: la reussite au long terme Aspects psychologiques et pharmacologiques", REVUE MEDICALE DE LIEGE, LIEGE, BE, vol. 61, no. 1, 1 January 2006 (2006-01-01), pages 27-30, XP003026378, ISSN: 0370-629X
- GARG S ET AL: "Development and evaluation of a buccal bioadhesive system for smoking cessation therapy", DIE PHARMAZIE, GOVI VERLAG PHARMAZEUTISCHER VERLAG GMBH, ESCHBORN, DE, vol. 62, no. 4, 1 January 2007 (2007-01-01), pages 266-272, XP003026379, ISSN: 0031-7144, DOI: 10.1691/PH.2007.4.6144
- DELVAUX M. ET AL: 'L'aide a l'arret du tabagisme: la reussite au long terme Aspects psychologiques et pharmacologiques' REVUE MEDICALE DE LIEGE vol. 61, no. 1, 2006, pages 27 - 30, XP003026378
- GARG S. ET AL: 'Development and evaluation of a buccal bioadhesive system for smoking cessation therapy' PHARMAZIE vol. 62, no. 4, 2007, pages 266 - 272, XP003026379
- GILLES PONCHEL: 'Formulation of oral mucosal drug delivery systems for the systemic delivery of bioactive materials' ADVANCED DRUG DELIVERY REVIEWS vol. 13, no. 1-2, 1994, pages 75 - 87, XP023861787

## Description

### Field of the Invention

The present invention relates to a multi portion intra-oral dosage form where at least one portion is rapidly disintegrating and at least one portion is slowly disintegrating, whereby the disintegration time for the slowest disintegrating portion is at least two times longer than for the most rapidly disintegrating portion. Of certain interest is use of sensory markers/signals as conceptual aids for the subject.

Also described are a method and a system for delivering active agents, such as nicotine and/or metabolites thereof, such as cotinine, nicotine N'-oxide, nornicotine, (S)-nicotine-N-β-glucuronide and mixtures, isomers, salts and complexes thereof as well as use and production of said formulations.

### Background of the Invention

Tobacco dependence and reduction thereof is a desirable goal. In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, and smokers characteristically display a strong tendency to relapse after having successfully stopped smoking for a time. Nicotine is the world's second most used drug, after caffeine from coffee and tea.

The main problem with tobacco smoking is its enormous implications on health. It is estimated that smoking related diseases cause some 3 - 4 million deaths per year. According to Centers for Disease Control and Prevention, cigarette smoking among adults - United States, 1995. MMWR 1997; 46:1217 - 1220 around 500,000 persons in USA die each year as a result of tobacco use. In fact, excessive smoking is now recognised as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug. The incidence of smoking is still rising in many countries, especially in less developed countries.

The most advantageous thing a heavy smoker can do is to stop smoking completely or at least to reduce his/her smoking. Experience shows, however, that most smokers find this extremely difficult since, mostly, tobacco smoking results in a dependence disorder or craving. The World Health Organization ("WHO") has in its International Classification of Disorders a diagnosis called Tobacco Dependence. Others like the American Psychiatric Association call the addiction Nicotine Dependence. It is generally accepted that these difficulties to stop smoking result from the fact that those heavy smokers are dependent on nicotine. The most important risk factors related to health are, however, substances that are formed during the combustion of tobacco, such as carcinogenic tar products, carbon monoxide, N-nitrosamines, aldehydes, and hydrocyanic acid.

### Effects of nicotine

Nicotine is an addictive poisonous alkaloid C₅H₄NC₄H₇NCH₃, derived from the tobacco plant. Nicotine is also used as an insecticide. The administration of nicotine (for example, in the form of smoking a cigarette, cigar or pipe) can give a pleasurable feeling to the smoker. However, smoking has health hazards and it is, therefore, desirable to formulate an alternative way of administering nicotine in a pleasurable and harmless manner that can be used to facilitate withdrawal from smoking and/or used as a replacement for smoking.

When smoking a cigarette, nicotine is quickly absorbed into the smoker's blood and reaches the brain within around ten seconds after inhalation. The quick uptake of nicotine gives the consumer a rapid satisfaction, or kick. The satisfaction usually lasts during the smoking time of the cigarette and for a period of time thereafter. The poisonous, toxic, carcinogenic, and addictive nature of smoking has provided strong motivation to develop methods, compositions and devices, which can be used to break the habit of smoking cigarettes.

### Nicotine replacement products.

One way to reduce smoking is to provide nicotine in a form or manner other than by smoking and some products have been developed to fulfil this need. Nicotine containing formulations are currently the dominating treatments for tobacco dependence. Formulations comprising nicotine metabolites, such as cotinine, nicotine N'-oxide, nornicotine, (S)-nicotine-N-β-glucuronide and mixtures thereof, with or without nicotine, have also been found useful for this purpose.

The successes in achieving reduction in the incidence of smoking have been relatively poor using presently known products. The present state of the art involves both behavioural approaches and pharmacological approaches. More than 80 % of the tobacco smokers who initially quit smoking after using some behavioural or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market. Several methods and means have been described for diminishing the desire of a subject to use tobacco, which comprises the step of administering to the subject nicotine or a derivative thereof as described in e g U.S. Patent Number 5,810,018 (oral nicotine-containing spray), U.S. Patent Number 5,939,100 (nicotine-containing micro spheres) and U.S. Patent Number 4,967,773 (nicotine-containing lozenge).

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Ways of administrating nicotine by way of delivering directly into the nasal cavity by spraying is known from U.S. Patent Number 4,579,858, DE 32 41 437 and WO93/12764. There may be local nasal irritation, however, with use of nasal nicotine formulations. The difficulty in administration also results in unpredictability of the dose of nicotine administered.

The use of skin patches for transdermal administration of nicotine has been reported (Rose, in Pharmacologic Treatment of Tobacco Dependence, (1986) pp. 158-166, Harvard Univ. Press). Nicotine-containing skin patches that are in wide use today can cause local irritation and the absorption of nicotine is slow and affected by cutaneous blood flow.

Also, inhaling devices resembling a cigarette are known for uptake of nicotine vapours as suggested in U.S. Patent Number 5,167,242. Said means and methods address the problems associated with addiction to nicotine.

One successful product that is used as a smoking substitute and/or as a smoking cessation aid and which is based on nicotine, is the chewing gum Nicorette^{®}. This product was one of the first nicotine replacement forms that was approved by the Food and Drug Administration (FDA) and is still one of the most used nicotine replacement products. Nicorette^{®} chewing gum has been on the market in about 60 countries for several years. In this chewing gum the nicotine is present in the form of a complex with an insoluble cation-exchanger (polacrilex) that is dispersed in a gum base. The nicotine is slowly released from the gum due to chewing and will reach similar plasma levels as when smoking a cigarette after about 30 minutes depending on the chewing technique, i e slow or active. Patents related to this product are e g U.S. Patent Number 3,877,468, U.S. Patent Number 3,901,248 and U.S. Patent Number 3,845,217.

Other successful nicotine replacement products are Nicorette® Microtab and its successor Microtab Lemon. These tablets are sublingual tablets and provides slow release of nicotine that aids a subject to achieve a nicotine plasma profile similar (bioequivalent) to that of the Nicorette® chewing gum.

Pharmaceuticals intended for oral administration are typically provided in solid form as tablets, capsules, pills, lozenges, or granules. Rapidly disintegrating tablets are often employed in the administration of pharmaceuticals where it is impractical to provide a tablet for swallowing whole, for instance with paediatric patients. Several workers in the field have explored rapidly disintegrative tablets (e g, U.S. Patent Nos. 6,106,861 and 6,024,981 and PCT Application No. WO 99/47126).

Applicant's invention relates for example to an intra-oral multi portion dosage form that combines the use of e g a rapidly disintegrating portion comprising a pharmaceutically active agent with a slower disintegrating hard portion, e g a lozenge. The dosage form, thus, may provide both the benefit of fast delivery of pharmaceutically active compound/s contained within the rapidly disintegrating portion with the benefit of slow/extended release from the slowly disintegrating portion that may comprise another or the same pharmaceutically active compound/s. The dosage form may be but are not limited to a lozenge, a tablet, a capsule, an oral film, a sublingual tablet, a troche, a lolly pop, a hard boiled candy, a chocolate lens, a micro bead, a jelly, a jelly bean, a semi solid, a center filled dosage form, a combination thereof or any other intra-oral dosage form.

Furthermore, the dosage form facilitates the use of sensory markers/signals or organoleptic sensations as a sensory aid to indicate to the subject the content of different layers, e g menthol or cinnamon in the rapidly disintegrating portion and evergreen mint flavour in the slowly disintegrating portion. In further embodiments it can also be envisaged that a sensory signal is conveyed to the subject e g when the pharmaceutically active compound/s has started /will start to be released there from or when approximately one-quarter of the active has been released etc.

### Prior art

US 5,879,710 discloses a specific mucoadhesive double layer formulation for administration of melatonin.

US 5,236,713 discloses a laminated preparation for intermittently releasing an active agent.

WO 1992/01445 discloses an osmotic device for controlled delivery of nicotine base through an oral mucosa membrane.

US 20060073189A1 discloses monolayer oral preparations for biphasic delivery of nicotine.

US 5,681,583 discloses a double-layer tablet to be swallowed for administration of an active material, whereby one layer releases the active quickly, while the other layer releases the active more gradually. A tablet to be swallowed is intended for uptake of an active in the GI tract, which is totally different from a dosage form for intraoral uptake of an active.

US 20030118648A1 discloses a pharmaceutical composition comprising a moulded triturate portion surrounded by a compressed annular tablet comprising a pharmaceutically active ingredient.

WO2001/037814 discloses a tablet that is attachable to the buccal mucosa, where it releases a substance in a multiphasic manner, typically with an initial burst release followed by controlled release over a longer period. '814 though does not comprise any proof of utility for this concept.

US 6,248,760 discloses a multi-layered nicotine-containing tablet where a non-toxic matrix layer comprises an antacid, but does not contain nicotine.

### Detailed Description of the Invention

### Definitions

The below definitions apply *mutatis mutandis* on expressions being similar to those being defined below.

The term "organoleptic sensation" is herein intended to mean a feature of the embodiment that is discernable to the taste, mouth feel, smell, hearing and/or vision of the subject such as, but not limited to, flavor, cooling, burning, warming, tingling, bubbling, foaming, effervescing, heating, mouth watering, crunchiness, stickiness, physical form, texture e g hardness, softness, roughness, and engravings.

The term "nicotine mimicking component" is herein intended to mean a component that in some respects may be considered to share or resemble any organoleptic feature of nicotine irrespective of the form of nicotine.

The term "intra-oral dosage form" is herein intended to mean dosage form intended for administration into the systemic blood circulation by means of absorption of an active principle, i.e. a pharmaceutically active compound, by any tissue of the oral cavity.

The term "oral formulation" or similar is herein intended to mean all formulations being suitable to be placed in the oral cavity for delivering nicotine essentially to the tissue of the oral cavity.

The term "complete reduction" or "complete" is herein intended to mean complete or substantially complete reduction.

The term "controlled release" is intended to mean a release of nicotine from an oral formulation in the oral cavity of the subject, whereby active sucking or other manipulation of the oral formulation is controlling the amount of nicotine released.

The term "disintegration" is intended to mean disintegration of a portion into particles and subsequent solubilization as well as dissolving of a portion or melting of a portion and the spreading of a liquid.

The term "portion" is intended to mean a separate entity of a dosage form. Examples of a portion is e.g. a tablet layer, a hard boiled candy layer, a melt layer, a film, a liquid, a capsule, a coating, and a wine gum.

The term "slow release" is intended to mean that e g nicotine is released from the oral formulation upon sucking or other manipulation over a period of time for example, several minutes to an hour.

The term "unit formula" is intended to mean one multi portion intra-oral formulation unit.

The term "transient" is intended to mean a non-permanent change, upon which the relevant state, e g biological or physiological state, after a certain period of time will return to its value or behaviour prior to said change.

The terms "buccal" and "buccally" are herein intended to pertain to all of or any part of the tissue of the oral cavity.

The term "coating" or "coating layer" or similar is here intended to mean a layer which totally encloses a solid or semi-solid object, e g a solid or semi-solid pharmaceutical dosage form. A multiportion intra-oral dosage form according to the present invention may or may not be coated.

### Summary of the Invention

The present invention relates to a multi portion intra-oral dosage form as described in claim 1 where at least one portion is rapidly disintegrating and at least one portion is slowly disintegrating, whereby the disintegration time for the slowest disintegrating portion is at least two times longer than for the most rapidly disintegrating portion.

Of certain interest is use of sensory markers/signals as conceptual aids for the subject, where at least one portion may comprises a component for creating an organoleptic sensation. Also contemplated are a method and a system for delivering nicotine and/or metabolites thereof, such as cotinine, nicotine N'-oxide, nornicotine, (S)-nicotine-N-β-glucuronide and mixtures, isomers, salts and complexes thereof as well as use and production of said formulations. Nicotine and/or metabolites thereof, such as cotinine, nicotine N'-oxide, nornicotine, (S)-nicotine-N-β-glucuronide and mixtures, isomers, salts and complexes thereof in any form and/or a nicotine-mimicking compound may be included in one or several portions of the dosage form.

An object of the present invention is thus to provide an efficient and effective product to deliver nicotine and/or metabolites thereof, such as cotinine, nicotine N'-oxide, nornicotine, (S)-nicotine-N-β-glucuronide and mixtures, isomers, salts and complexes thereof and/or a nicotine-mimicking compound and optionally component/components for creating an organoleptic sensation to a subject so as to obtain a transmucosal uptake of nicotine and/or metabolites thereof, such as cotinine, nicotine N'-oxide, nornicotine, (S)-nicotine-N-β-glucuronide and mixtures, isomers, salts and complexes thereof in the oral cavity of the subject. Therefore, the present invention provides the dosage form according to claim 1. Thus, described herein is a method for delivering for example nicotine and/or metabolites thereof, such as cotinine, nicotine N'-oxide, nornicotine, (S)-nicotine-N-β-glucuronide and mixtures, isomers, salts and complexes thereof in any form to a subject comprising administering to a subject an oral formulation containing nicotine and/or metabolites thereof, such as cotinine, nicotine N'-oxide, nornicotine, (S)-nicotine-N-β-glucuronide and mixtures, isomers, salts and complexes thereof in any form into the oral cavity of the subject and if needed allowing the nicotine and/or metabolites thereof, such as cotinine, nicotine N'-oxide, nornicotine, (S)-nicotine-N-β-glucuronide and mixtures, isomers, salts and complexes thereof in any form in the oral formulation to be released in the saliva in the oral cavity and absorbed into the systemic circulation of the subject as well as a method for producing said oral formulation.

Also described is a method for obtaining reduction of the urge to smoke or use tobacco containing material and/or for providing a sense of smoking satisfaction without smoking, comprising the steps of replacing at least partly the tobacco containing material with the above said oral formulation, administering to a subject an oral formulation containing nicotine and/or metabolites thereof, such as cotinine, nicotine N'-oxide, nornicotine, (S)-nicotine-N-β-glucuronide and mixtures, isomers, salts and complexes thereof in any form into the oral cavity of the subject and if needed allowing the nicotine and/or metabolites thereof, such as cotinine, nicotine N'-oxide, nornicotine, (S)-nicotine-N-β-glucuronide and mixtures, isomers, salts and complexes thereof in any form of the oral formulation to be released in the saliva in the oral cavity and absorbed by the subject.

Also described is a system for delivering nicotine and/or metabolites thereof, such as cotinine, nicotine N'-oxide, nornicotine, (S)-nicotine-N-β-glucuronide and mixtures, isomers, salts and complexes thereof in any form to a subject, comprising said oral formulation and at least one other means for obtaining reduction of the urge to smoke or use of tobacco as well as a system for obtaining reduction of the urge to smoke or otherwise use tobacco and/or for providing a sense of smoking satisfaction without smoking, comprising an oral formulation as described above and at least one other method for obtaining reduction of the urge to smoke or otherwise use tobacco. Said system may be a system wherein the at least other method is selected from the group consisting of administration of nicotine and/or metabolites thereof, such as cotinine, nicotine N'-oxide, nornicotine, (S)-nicotine-N-β-glucuronide and mixtures, isomers, salts and complexes thereof in any form to a subject through for example, but not limiting to, mouth sprays, nasal sprays, transdermal patches, inhaling devices, lozenges, tablets and parenteral methods, subcutaneous methods, and transmucousal methods; or other use of tobacco.

In addition, the production of a formulation comprising nicotine and/or metabolites thereof, such as cotinine, nicotine N'-oxide, nomicotine, (S)-nicotine-N-β-glucuronide and mixtures, isomers, salts and complexes thereof in any form for use in therapy is described, wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight control.

Other features and advantages of the present invention will be apparent from the detailed description of the invention and from the claims.

### Pharmaceutically Active Agent

The pharmaceutically active agent included within the rapidly disintegrating portion/s or within the slowly disintegrating portion/s may be a smoking cessation compound such as, but not limited to, nicotine and/or metabolites thereof, such as cotinine, nicotine N'-oxide, nornicotine, (S)-nicotine-N-β-glucuronide and mixtures, isomers, salts and complexes thereof in any form, varenicline, bupropion, nortriptyline, doxepin, fluoxetine, imipramine, moclobemide, and/or cytisine and pharmaceutically acceptable salts, inclusion complexes and prodrugs thereof. +

The one or more pharmaceutically active agent(s) may also be chosen from
o the antiinflammatory agents diclofenac, ketorolac, indometacin, tornoxicam, piroxicam, tenoxicam, ketoprofen, celecoxib and roficoxib;
o the muscle relaxants orphenadrine and baclofen;
o the drugs affecting bone mineralization alendronic acid and risedronic acid;
o the analgesics propoxyphene, buprenorfin, ketobenidon, hydromorphone, tramadol, morphine, and tapentadol;
o the antimigraine preparations: dihydroergotamine, ergotamine, eletriptan, naratriptan, rizatriptan, sumatriptan and zolmitriptan;
o the anti-Parkinson drugs pramipexole, ropinirole and selegiline;
o the anxiolytics alprazolam, diazepam, lorazepam and oxazepam;
o the hypnotics flunitrazepam, midazolam, nitrazepam, triazolam, zaleplone, zopiclone, zolpiderm, clometiazole and propiomazine;
o the psychostimulant caffeine;
o the drugs against substance dependence bupropione, lobeline, naltrexone and methadone;
o the gastric ulcer remedy famotidine;
o the antispasmodic hyoscyamine;
o the antiemetics metoclopramide, ondansetron, scopolamine, hyoscine, perfenazine, procloperazine and haloperidol;
o the antidiabetic agent rosiglitazone;
o the cardiovascular agents etilefrin, glyceryl trinitrate, isosorbide dinitrate and isosorbide mononitrate;
o the antihypertensive agent hydralazine;
o the diuretics furosemide and amiloride;
o the beta-receptor blocking agents propranolol and timolol;
o the calcium channel blocker amlodipine;
o the ACE-inhibitors kaptopril, lisinopril and fosinopril;
o the serum lipid reducing agent simvastatin;
o the antipsoriatic acitretin;
• the antiasthmatic terbutaline;
• the antitussives codeine and noscapine;
• the antihistamines clemastine, chlorpheniramine, cyproheptadine, loratadine and acrivastine: the antidepressant and anti-sexual dysfunction drug dapoxetine;
• the anti-sexual dysfunction drugs sildenafil (Viagra), tadalafil, vardenafil, cabergoline and pramipexole,
• the antiepileptic topiramate, and
• the oral and/or gastrointestinal and/or general health promoting agent Lactobacillus reuteri.
where the therapeutic area given shall be regarded as a non-limiting example of a suitable therapeutic area for the stated drug(s).

In the present invention the dual portion lozenge drug delivery system may be used for delivering nicotine and/or metabolites thereof, such as cotinine, nicotine N'-oxide, nornicotine, (S)-nicotine-N-β-glucuronide and mixtures, isomers, salts and complexes thereof to a subject for treating e g tobacco dependence. The drug delivery system provides a potentially advantageous drug delivery system for delivery of nicotine and/or metabolites thereof, such as cotinine, nicotine N'-oxide, nornicotine, (S)-nicotine-N-β-glucuronide and mixtures, isomers, salts and complexes thereof, where the rapidly disintegrating portion facilitates a rapid release of nicotine and/or metabolites thereof, such as cotinine, nicotine N'-oxide, nornicotine, (S)-nicotine-N-β-glucuronide and mixtures, isomers, salts and complexes thereof in the saliva in the oral cavity and subsequent absorption into the systemic circulation of a subject followed by a prolonged release and absorption into the systemic circulation from the slower disintegrating portion/s. A number of nicotine replacement forms are available but the present drug delivery system provides new means for producingsmoking cessation products and increasing the compliance and potentially also reducing the initial nicotine craving as well as the craving over time and hence reducing the urge to use tobacco-containing material.

The portion/s may also comprise for example, but not limited to, zinc, chlorhexidine, L. reuteri, nystatin, amphotericin, miconazole, phenylephrine, dextromethorphan, pseudoephedrine, acetaminophen, ibuprofen, ketoprofen, loperamide, famotidine, calcium carbonate, simethicone, pseudoephedrine, chlorpheniramine, methocarbomal, chlophedianol, ascorbic acid, menthol, thymol, methyl salicylate and eucalyptol, pectin, dyclonine, benzocaine, and pharmaceutically acceptable salts and derivatives thereof.

### Nicotine

With nicotine it is intended to include nicotine, 3-(1-methyl-2-pyrrolidinyl)-pyridine, with its base form, including synthetic nicotine as well as nicotine extracts from tobacco plants, or parts thereof, such as the genus Nicotiana alone or in combination; or pharmaceutically acceptable salts, inclusion complexes, isomers and prodrugs thereof.

In preferred embodiments, the nicotine in any form is selected from the group consisting of the free base form of nicotine, a nicotine salt, a nicotine derivative, such as a nicotine cation exchanger, a nicotine inclusion complex or nicotine in any non-covalent binding, nicotine bound to zeolites, nicotine bound to cellulose or starch micro spheres, and mixtures thereof.

Numerous nicotine salts are known, and may be used, e g the salts presented in Table 1, preferably monotartrate, hydrogen tartrate (also called bitartrate or bitartrate dihydrate), citrate, malate, and/or hydrochloride.

**Table 1. Examples of possible acids useful for nicotine salt formation**

| Acid | Molar ratio* of acid:nicotine |
|---|---|
| Formic | 2:1 |
| Acetic | 3:1 |
| Propionic | 3:1 |
| Butyric | 3:1 |
| 2-Methylbutyric | 3:1 |
| 3-Methylbutyric | 3:1 |
| Valeric | 3:1 |
| Lauric | 3:1 |
| Palmitic | 3:1 |
| Tartaric | 2:1 |
| Citric | 2:1 |
| Malic | 2:1 |
| Oxalic | 2:1 |
| Benzoic | 1:1 |
| Gentisic | 1:1 |
| Gallic | 1:1 |
| Phenylacetic | 3:1 |
| Salicylic | 1:1 |
| Phthalic | 1:1 |
| Picric | 2:1 |
| Sulfosalicylic | 1:1 |
| Tannic | 1:5 |
| Pectic | 1:3 |
| Alginic | 1:2 |
| Hydrochloric | 2:1 |
| Chloroplatinic | 1:1 |
| Silicotungstic | 1:1 |
| Pyruvic | 2:1 |
| Glutamic | 1:1 |
| Aspartic | 1:1 |

| | |
|---|---|
| * recommended at the time of production | |

The inclusion complex may include cyclodextrin complexation, such as complexation of the active pharmaceutically compound with cyclodextrin where preferably the cyclodextrin used is chosen among α-, β- and γ-cyclodextrin, the hydroxypropyl derivatives of α-, β- and γ-cyclodextrin, sulfoalkylether cyclodextrins such as sulfobutylether β-cyclodextrin, alkylated cyclodextrins such as the randomly methylated β-cyclodextrin, and various branched cyclodextrins such as glucosyl- and maltosyl-β-cyclodextrin.

Some suitable cation exchangers are given in below Table 2 and are further disclosed in U.S. 3,845,217. Preferred are nicotine cation exchangers of polyacrylates, such as the Amberlite collection from Rohm & Haas.

**Table 2 Examples of cation exchangers**

| Name | Type of crosslinked polymer | Manufacturer |
|---|---|---|
| Amberlite IRC 50 | Divinylbenzene-methacrylic acid | Rohm & Haas |
| Amberlite IRP 64 | Divinylbenzene-methacrylic acid | Rohm & Haas |
| Amberlite IRP 64M | Divinylbenzene-methacrylic acid | Rohm & Haas |
| BIO-REX 70 | Divinylbenzene-acrylic acid | BIO-RAD Lab. |
| Amberlite IR 118 | Styrene-divinylbenzene | Rohm & Haas |
| Amberlite IRP 69 | Styrene-divinylbenzene | Rohm & Haas |
| Amberlite IRP 69M | Styrene-divinylbenzene | Rohm & Haas |
| BIO-REX 40 | Phenolic | BIO-RAD Lab. |
| Amberlite IR 120 | Styrene-divinylbenzene | Rohm & Haas |
| Dowex 50 | Styrene-divinylbenzene | Dow Chemical |
| Dowex 50W | Styrene-divinylbenzene | Dow Chemical |
| Duolite C 25 | Styrene-divinylbenzene | Chemical Process Co |
| Lewatit S 100 | Styrene-divinylbenzene | Farbenfabriken Bayer |
| Ionac C 240 | Styrene-divinylbenzene | Ionac Chem. |
| Wofatit KP S 200 | Styrene-divinylbenzene | I.G. Farben Wolfen |
| Amberlyst 15 | Styrene-divinylbenzene | Rohm & Haas |
| Duolite C-3 | Phenolic | Chemical Process |
| Duolite C-10 | Phenolic | Chemical Process |
| Lewatit KS | Phenolic | Farbenfabriken Bayer. |
| Zerolit 215 | Phenolic | The Permutit Co. |
| Duolite ES-62 | Styrene-divinylbenzene | Chemical Process |
| BIO-REX 63 | Styrene-divinylbenzene | BIO-RAD Lab. |
| Duolite ES-63 | Styrene-divinylbenzene | Chemical Process |
| Duolite ES-65 | Phenolic | Chemical Process |
| Ohelex 100 | Styrene-divinylbenzene | BIO-RAD Lab. |
| Dow Chelating Resin A-1 | Styrene-divinylbenzene | Dow Chemical Company |
| CM Sephadex C-25 | Dextran | Pharmacia Fine Chemicals |
| SE Sephadex C-25 | Dextran | Pharmacia Fine Chemicals |

### Amount and distribution of the nicotine in the oral formulation

The term nicotine is below intended to include nicotine metabolites, such as cotinine, nicotine N'-oxide, nornicotine, (S)-nicotine-N-β-glucuronide and mixtures, isomers, salts and complexes thereof unless the context indicates that just nicotine as such is meant,

The nicotine in any form according to the invention is formulated to provide the subject with a dose to achieve an effect. The effect may be to provide a sense of smoking satisfaction without smoking. Another effect of the administered nicotine in any form may be a reduction of the urge to smoke or use tobacco.

The effect may also be a combination of reduction of said urge and providing a sense of smoking satisfaction without smoking. The amount of the nicotine should be sufficient to provide such an effect in a subject. This amount may, of course, vary from person to person.

According to the invention, embodiments of the oral formulation comprise embodiments wherein nicotine in any form is present in an amount of 0.05 - 12 mg calculated as the free base form of nicotine per unit dose of the oral formulation. This may in different embodiments include 0.05, 0.5, 1, 2, 3, 4, 5, 6, 7, 8, 9, 10 , 11 or 12 mg calculated as the free base form of nicotine per unit dose, preferably in an amount of 0.1 - 6 mg, more preferably in an amount of 1 - 6 mg, and most preferably in an amount of 2 - 5 mg calculated as the free base form of nicotine per unit dose.

The nicotine in any form may be distributed in the oral formulations in different embodiments. Different distributions of the nicotine throughout the oral formulations will imply administration of the nicotine to the subject in different ways. This may, then, provide several possibilities to adjust the composition of the oral formulation according to different needs of different subjects depending on the urge to smoke or use tobacco of the subject. In the below Examples are disclosed different such embodiments.

### Buffering agents

The rapidly disintegrating portion(s) and/or the slowly disintegrating portion(s) may also comprise a suitable system of buffering agent/s to facilitate nicotine administration. Absorption of nicotine from the oral cavity to the systemic circulation is dependent on the pH of the saliva, pH of the blood plasma and the pKa of nicotine, which is about 7.8. Thus, the level and type of buffering agent/s or combination thereof will affect the pH of the saliva and hence the absorption of nicotine in a free base form, which is the form predominantly absorbed through the mucosa. The buffering is designed so as to achieve a transient buffering of the saliva of a subject during melting, disintegration or dissolution of the oral formulation. As the change is transient, the pH will return to its normal value after a certain period of time.

The buffering agent may be but are not limited to buffering agents from the group consisting of carbonate (including bicarbonate or sesquicarbonate), trometamol (2-amino-2-hydroxymethyl-1,3-propanediol, and also referred to as tromethamine, tris(hydroxymethyl aminomethane and TRIS), glycinate, different phosphate systems such as trisodium phosphate, disodium hydrogen phosphate; and tripotassium phosphate, dipotassium hydrogen phosphate, glycerophosphate or citrate of an alkali metal (such as potassium or sodium, or ammonium), e g trisodium and tripotassium citrate, different hydroxides, amino acids, e g as per below Table 3, and mixtures thereof.

**Table 3 Examples of useful amino acids.**

| **compound** | **CAS number** | **pKa value (in interval 8 - 9,6)** | **Solubility in water, g/kg** |
|---|---|---|---|
| Arginine | 74-79-3 | 9,00 | 182,6^{a)} |
| Aspargine | 70-47-3 | 8,73 | 25,1 |
| Glutamic acid | 56-86-0 | 9,58 | 8,61 ^{a)b)} |
| Glutamine | 56-85-9 | 9,00 | 42 |
| Glycine | 56-40-6 | 9,58 | 250,9 |
| Histidine | 71-00-1 | 9,09 | 43,5 |
| Isoleucine | 73-32-5 | 9,60 | 34,2 |
| Leucine | 61-90-5 | 9,58 | 22,0 |
| Lysine | 56-97-1 | 9,16 | Very soluble ^{a)b)} |
| Methionine | 63-68-3 | 9,08 | 56 |
| Phenylalanine | 63-91-2 | 9,09 | 27,9 |
| Serine | 56-45-1 | 9,05 | 50,2 |
| Threonine | 72-19-5 | 8,96 | 98,1 |
| Valine | 72-18-4 | 9,52 | 88,5 |
| Cysteic acid | 13100-82-8 | 8,70 | Very soluble |
| N-Glycylglycine | 556-50-3 | 8,10 | No information |
| Ornithine | 70-26-8 | 8,78 | Very soluble |

| | | | |
|---|---|---|---|
| a) reported as buffer in non-nicotine-containing pharmaceutical formulations. b) low or uncertain value on solubility in water. | | | |

The captioned data on the amino acids are taken from "Handbook of Chemistry and Physics", 85th edition; Table 7-1 ("20 standard amino acids that are the basic constituents of proteins") and Table 7-2 ("Amino acids and related compounds of biochemical importance").

### Other additives to the oral formulation

Other additives may be added optionally to the oral formulation. Optional additives comprise at least one or more additives selected from the group consisting of solvents, such as ethanol and water; co-solvents, such as propylene glycol; stabilisers, such as preservatives, e g antioxidants; softeners, such as sorbitol and glycerine; thickening agents, such as colloidal silicon dioxide; binding agents, such as xanthan gum; filling agents, such as mannitol, isomalt, cocoa powder and Crospovidone; solubilizers, such as Polysorbat 80 and Atmos 300; rubbers, lipid barriers, such as sucrose fatty acid esters and hydrogenated vegetable oils; film forming agents, such as porcine gelatine, Pullulan, carrageenan, pectin, locust bean gum and xanthan gum; emulsifiers, such as pectin, soy lecithin, glycerol monostearate, castor oil and poloxamer; glidants, such as colloidal silicon dioxide; lubricants, such as magnesium stearate; coating agents, such as castor oil and sorbitol; melting vehicles, such as vegetable oils; sweeteners, flavors, aromatics, cooling agents, enhancers, colouring agents, vitamins, minerals, fluorine, breath fresheners, tooth whitening agents and mixtures thereof. According to the invention, at least one of such additives is optionally added to the product.

Enhancers may be added essentially to increase the transmucosal uptake of nicotine from the oral cavity.

Sweeteners are added essentially to improve the taste. Sweeteners comprise one or more synthetic or natural sugars, i e any form of carbohydrates suitable for use as sweetener, as well as so called artificial sweeteners such as saccharin, sodium saccharin, aspartame, e g NutraSweet^{®}, acesulfame or Acesulfame K, potassium acesulfame, thaumatin, glycyrrhizin, sucralose, dihydrochalcone, alitame, miraculin, monellin, stevside and neotame.

Suitable sweeteners may be selected from the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, erythritol, maltitol syrup (or hydrogenated starch hydrolyzate), isomalt, lactitol; and mixtures of sugars including glucose syrup, e g starch hydrolysates, containing a mixture of dextrose, maltose and a range of complex sugars, invert sugar syrup, e g sucrose inverted by invertase (also called sucrase or sacchrase) containing a mixture of dextrose and fructose, high sugar content syrups such as treacle and honey containing a mixture of particular leavulose, dextrose, maltose, lactitole, sucrose, resins, dextrin and higher sugars; and malt or malt extracts.

The flavor and aroma additives may comprise one or more synthetic or natural taste-masking, flavoring or aromatizing agents and may be added as liquids and/or as powder. Flavor and aroma agents may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavor of the fruit, e g strawberry, raspberry and black currant; artificial and natural flavors of brews and liquors, e g cognac, whisky, rum, gin, sherry, port, and wine; tobacco, coffee, tea, cocoa, and mint; fruit juices including expelled juice from washed, scrubbed fruits such as lemon, orange, and lime; spear mint, pepper mint, wintergreen, cinnamon, cacoe/cocoa, vanilla, liquorice, menthol, eucalyptus, aniseeds, nuts (e g peanuts, coconuts, hazelnuts, chestnuts, walnuts, colanuts), almonds, raisins; and powder, flour, or vegetable material parts including tobacco plant parts, e g genus Nicotiana, in amounts not contributing significantly to the level of nicotine, and ginger.

Colouring additives may be selected from dyes being approved as a food additive.

Stabilizing additives may be selected from the group consisting of antioxidants including vitamin E, i e tocopherole, ascorbic acid, sodium pyrosulfite, butylhydroxytoluene, butylated hydroxyanisole, edetic acid and edetate salts ; and preservatives including citric acid, tartaric acid, lactic acid, malic acid, acetic acid, benzoic acid, and sorbic acid. Preferred embodiments comprise an antioxidant as the stabiliser, and even more preferably the antioxidant vitamin E and/or butylated hydroxytoluene (BHT).

### Compressible Excipients

In one embodiment, at least one rapidly disintegrating tablet portion includes one or more compressible excipients. In one embodiment the at least one rapidly disintegrating intra-oral tablet portion comprises at least 40% by weight of such compressible excipients. With "compressible excipient" is here meant an ingredient that can be compressed into a tablet shape without the addition of other binding agents. In certain embodiments, the compressible excipient is in the form of a hydrate, and may be selected from organic compounds such as dextrose monohydrate, maltodextrin, lactose monohydrate, and dextrin, as well as inorganic compounds including dibasic calcium phosphate dihydrate, dibasic sodium phosphate dihydrate, dibasic sodium phosphate heptahydrate, dibasic sodium phosphate dodecahydrate, monobasic sodium phosphate monohydrate and monobasic sodium phosphate dihydrate. In one embodiment, the rapidly disintegrating tablet portion includes a compressible excipient selected from the group consisting of isomalt, dextrose monohydrate, maltodextrin, lactose monohydrate, dextrin, mannitol, lactitol, sorbitol, xylitol, erythritol, sucrose, and lactose.

In one embodiment, the compressible excipient(s) are in the form of particles having an average particle diameter of from about 50 to about 500 microns, such as from about 75 to about 400 microns.

In one embodiment, the rapidly disintegrating tablet portion includes from about 5 to about 90 percent, such as from about 15 to about 75 percent, by weight of one or more compressible excipients. In one embodiment, the disintegrative tablet portion includes at least 40 percent by weight of the one or more compressible excipients, based on the total weight of the disintegrative tablet portion.

### Water-Swellable Excipients

In the present invention, the rapidly disintegrating tablet portion further includes one or more water-swellable excipients as defined in the claims. With "water swellable excipient" is here meant a material that is designed to swell or wick liquid upon contact with a liquid medium and to aid in the disintegration of the compressed tablet. The water-swellable excipient may be selected from superdisintegrants such as crospovidone, croscarmellose, sodium starch glycolate, cellulose compounds such as microcrystalline cellulose, starches, alginic acid and inorganic clays such as bentonite, attapulgite, and magnesium aluminum silicate. In one embodiment, the water-swellable excipient is at least partially hydrated and selected from the group consisting of sodium starch glycolate, crospovidone, croscarmellose, microcrystalline cellulose, starches, hydroxypropyl cellulose, and alginic acid.

In one embodiment, the amount of water-swellable excipient(s) in the rapidly disintegrating tablet portion is from about 0.1 to about 5 percent by weight, such as from about 0.5 to about 3 percent by weight of the total weight of the rapidly disintegrating tablet portion.

In one embodiment, the compressible excipient(s) is present in a greater amount than the water-swellable excipient(s). In one embodiment, the ratio of compressible excipient(s) to water-swellable excipient(s) in the disintegrative tablet portion is from about 1:1 to about 150:1, such as from about 10:1 to about 100:1, such as from about 25:1 to about 75:1.

### Effervescent Couple

In one embodiment, the disintegrative tablet portion further includes one or more effervescent couples. In one embodiment, effervescent couple includes one member from the group consisting of sodium bicarbonate, potassium bicarbonate, calcium carbonate, magnesium carbonate, sodium carbonate and one member selected from the group consisting of citric acid, malic acid, fumaric acid, tartaric acid, phosphoric acid, alginic acid.

In one embodiment, the combined amount of the effervescent couple(s) in the disintegrative tablet portion is from about 0.1 to about 20 percent by weight, such as from about 2 to about 10 percent by weight of the total weight of the disintegrative tablet portion.

### Additional information on ingredients

A rapidly disintegrating tablet portion may include conventional ingredients, including other fillers, which include water-soluble compressible carbohydrates such as dextrose, sucrose, Mannitol, sorbitol, maltitol, xylitol, lactose, and mixtures thereof; other conventional dry binders like polyvinyl pyrrolidone and the like; sweeteners such as aspartame, acesulfame potassium, sucralose, and saccharin; lubricants, such as magnesium stearate, stearic acid, talc, and waxes; preservatives; flavors; disintegrants, antioxidants; acidulants, such as but not limited to citric acid, malic acid, tartaric acid, ascorbic acid, and fumaric acid; surfactants; and coloring agents

A slowly disintegrating portion or portions may comprise an excipient selected from, but not limited to, the group consisting of isomalt, sucrose, dextrose, dextrose monohydrate, corn syrup, lactitol, lycasin, mannitol, sorbitol, erythritol, xylitol, starches, gelatinized starches, maltodextrin, lactose, lactose monohydrate, dextrin, and mixtures and/or derivatives thereof. The slowly disintegrating portion/s may comprise an excipient selected from but not limited to the group consisting of isomalt, sucrose, dextrose, corn syrup, lactitol, and lycasin, and mixtures and/or derivatives thereof.

Especially the rapidly disintegrating portion/s may comprise an effervescent couple comprising e g one member selected from the group consisting of sodium bicarbonate, potassium bicarbonate, calcium carbonate, magnesium carbonate, and sodium carbonate and one member selected from the group consisting of citric acid, malic acid, fumaric acid, tartaric acid, and alginic acid.

### Examples

The skilled person may on the basis of the following examples envisage also other embodiments of the present invention. Batch sizes for the manufacture of the below formulations may be modified according to the actual need and to the actual production facilities.

### Example 1

Preparation of a dual portion tablet where the rapidly disintegrating portion contains 0.5 mg nicotine (NRC) together with menthol flavor and the slowly disintegrating portion contains 1.5 mg nicotine (NRC) with a lemon flavor

### Method

The ingredients listed in below Table A 1 and Table A2 are sieved and thereafter blended, each separately, according to methods known in the art e g using a double cone blender. The two portions of blended material are then compressed into tablets by means of direct compression. The powder compression may for example be performed using a double-sided rotary tablet press with individual fill stations and where each of the two layers, i.e. the rapidly disintegrating tablet portion and the slowly disintegrating portion, are subjected to pre-compression and main compression, respectively, to form a dual portion lozenge.

**Table A1: Components of the rapidly disintegrating tablet portion.**

| Ingredients | Percent (w/w) | Mg/portion |
|---|---|---|
| Nicotine resin complex (20% nicotine) | 0.625 | 2.5* |
| Crospovidone | 0.75 | 3 |
| Microcrystalline Cellulose (Avicel PH100) | 5 | 20 |
| Dextrose Monohydrate | 90.74 | 362.96 |
| Trometamol | 1.875 | 7.5 |
| Menthol | 0.25 | 1 |
| Coloring agent | 0.01 | 0.04 |
| Magnesium Stearate | 0.75 | 3 |
| TOTAL | 100.0 | 400 |

| | | |
|---|---|---|
| * Equivalent to 0.5 mg dose of nicotine. | | |

**Table A2: Components of the slowly disintegrating portion.**

| Ingredients | Percent (w/w) | mg/portion |
|---|---|---|
| Nicotine resin complex (20% nicotine) | 0.75 | 7.5 |
| Sorbitol | 95.75 | 957.5 |
| Trometamol | 1.0 | 10 |
| Sodium Carbonate | 0.5 | 5 |
| Lemon flavor | 1 | 10 |
| Magnesium Stearate | 1 | 10 |
| TOTAL | 100.0 | 1000.0 |

| | | |
|---|---|---|
| * Equivalent to 1.5 mg Dose of nicotine. | | |

### Example 2

Preparation of a dual portion tablet where the slowly disintegrating portion has a rough geometric pattern or form or shape and the rapidly disintegrating portion has a smooth surface.

### Method

The same method as in Example 1, but for the shape of the punches used.

**Table B1: Components of the rapidly disintegrating portion.**

| Ingredients | Percent (w/w) | mg per tablet |
|---|---|---|
| Nicotine bitartrate dihydrate | 0.77 | 3.08* |
| Crospovidone | 0.75 | 3 |
| Microcrystalline Cellulose (Avicel PH100) 100101) | 5 | 20 |
| Dextrose Monohydrate | 85.32 | 345.28 |
| L-Arginine | 5.4 | 21.6 |
| Lemon | 1 | 4 |
| Coloring agent | 0.01 | 0.04 |
| Magnesium Stearate | 0.75 | 3 |
| TOTAL | 100.0 | 400 |

| | | |
|---|---|---|
| * Equivalent to a 1.0 mg dose of nicotine. | | |

**Table B2: Components of the slowly disintegrating portion.**

| Ingredients | Percent (w/w) | Mg/hard candy portion |
|---|---|---|
| Nicotine resin complex (20% nicotine) | 1 | 10* |
| Isomalt | 91.68 | 926.8 |
| L-Arginine | 4.32 | 43.2 |
| Mint | 1 | 10 |
| Magnesium Stearate | 1 | 10 |
| TOTAL | 100.0 | 1000.0 |

| | | |
|---|---|---|
| * Equivalent to a 2.0 mg dose of nicotine. | | |

### Example 3

Preparation of a dual portion tablet where the tablet upon contact with the saliva shows that the rapidly disintegrating portion is softer and may be experienced as flaky/crumbly as it disintegrates and the slowly disintegrating portion is harder and do not crumble/flake.

### Method

The same method and the same formulation as in Example 1 and 2 are used, but without added flavor. Hereby the difference in flakiness/crumbliness between the portions becomes more noticeable than in Examples 1 and 2.

### Example 4

Preparation of a triple portion tablet with two rapidly disintegrating portions, where one portion comprises 1 mg nicotine and the other portion comprises cinnamon flavor, and one slowly disintegrating portion, which comprises 3 mg nicotine.

### Method

Manufacturing principles according to the preceding examples are used.

**Table C1: Components of the first rapidly disintegrating tablet portion containing 1.0 mg nicotine.**

| Ingredients | Percent (w/w) | mg/portion |
|---|---|---|
| Nicotine resin complex (20% nicotine) | 1.25 | 5* |
| Crospovidone | 0.75 | 3 |
| Microcrystalline Cellulose (Avicel PH100) | 5 | 20 |
| Dextrose Monohydrate | 91.345 | 360.46 |
| Trometamol) | 1.875 | 7.5 |
| Menthol | 0.25 | 1 |
| Coloring agent | 0.01 | 0.04 |
| Magnesium Stearate | 0.75 | 3 |
| TOTAL | 100.0 | 400 |

| | | |
|---|---|---|
| * Equivalent to 1.0 mg dose of nicotine. | | |

**Table C2: Components of the second rapidly disintegrating tablet portion containing cinnamon flavor.**

| Ingredients | Percent (w/w) | mg/portion |
|---|---|---|
| Crospovidone | 0.75 | 3 |
| Microcrystalline Cellulose (Avicel PH100) | 5 | 20 |
| Dextrose Monohydrate | 91.345 | 360.46 |
| Trometamol | 1.875 | 7.5 |
| Cinnamon | 1.5 | 6 |
| Coloring agent | 0.01 | 0.04 |
| Magnesium Stearate | 0.75 | 3 |
| TOTAL | 100.0 | 400 |

**Table C3: Components of the slowly disintegrating portion.**

| Ingredients | Percent (w/w) | mg/portion |
|---|---|---|
| Nicotine resin complex (20% nicotine) | 1.5 | 15 |
| Sorbitol | 96.5 | 965 |
| Trometamol | 1.0 | 10 |
| Sodium Carbonate | 0.5 | 5 |
| Lemon flavor | 1 | 10 |
| Magnesium Stearate | 1 | 10 |
| TOTAL | 100.0 | 1000.0 |

| | | |
|---|---|---|
| * Equivalent to 3.0 mg dose of nicotine. | | |

### Example 5

Preparation of a double portion tablet as in Example 1, but where a pre-compressed slowly disintegrating portion has the shape of a torus in which the powder of the rapidly disintegrating portion is filled where after main compression is performed.

### Example 6

Preparation of a double portion tablet with 2 mg nicotine containing a slowly disintegrating boiled sugar portion and a rapidly disintegrating tablet portion.

### Method

The method for preparing the slowly disintegrating boiled sugar portion is as follows: Sieve the dry materials given in above Table D1. Add purified water, isomalt and maltitol solution to a stainless steel beaker. Mix and heat to ca 170°C during continuous mixing until the water is evaporated. Discontinue heating and cool to 135-140 °C. Add nicotine bitartrate dihydrate and mix until fully dispersed. Add buffer components and mix at 120 °C until dispersed thereafter add flavor and mix until uniform. While in the flowable state, deposit the hard candy portion blend into a circular stainless steel molds with dual flat faces. The resulting boiled sugar portion is allowed to cool and harden at room temperature for approximately 15 minutes. The hard candy portion is then placed into a rubber mold. Approximately 30 milligrams of powdered polyethylene glycol (PEG) 3350 is evenly dispersed along one surface of the hard candy portion.

**Table D1: Components of the slowly disintegrating boiled sugar portion blend.**

| Ingredients | Percent (w/w) | mg/hard candy portion |
|---|---|---|
| Nicotine bitartrate dihydrate (32.55% Nicotine) | 0.538 | 5.376* |
| Isomalt | 76.46 | 764.6 |
| Maltitol 75 % solution | 19.5 | 195 |
| Sodium carbonate anhydrous | 1 | 10 |
| Sodium Bicarbonate | 0.5 | 5 |
| Flavoring agents | 2 | 20 |
| Purified water | - | - |
| TOTAL | 100.0 | 1000.0 |

| | | |
|---|---|---|
| *Equivalent to a 1.75 mg does of nicotine. | | |

A flat-faced compressed tablet is manufactured according to Example 1 with components as per below Table D2.

**Table D2: Components of the rapidly disintegrating tablet portion.**

| Ingredients | Percent (w/w) | mg per tablet |
|---|---|---|
| Nicotine bitartrate dihydrate (32.55% Nicotine) | 0.192 | 0.768* |
| Crospovidone | 0.75 | 3 |
| Microcrystalline Cellulose (Avicel PH 100) 100101) | 5 | 20 |
| Dextrose Monohydrate | 88.8 | 355.19 |
| Sodium carbonate anhydrous | 2.5 | 10 |
| Flavoring agent | 2 | 8 |
| Coloring agent | 0.01 | 0.04 |
| Magnesium Stearate | 0.75 | 3 |
| TOTAL | 100.0 | 400 |

| | | |
|---|---|---|
| * Equivalent to a 0.25 mg dose of nicotine. | | |

The rapidly disintegrating tablet portion is adjoined to the boiled hard candy portion as follows: A flat-faced compressed tablet as per above is placed on top of the hard candy portion, and the resulting dosage form is placed into an oven providing a temperature being so high that the PEG 3350 melts and creates an adhesion between the compressed tablet and the hard candy portion. The resulting dual portion tablet is then allowed to cool at room temperature for 30 minutes and removed from the rubber mold.

### Example 7

Preparation of a double portion tablet with 2 mg nicotine, containing a slowly disintegrating hard boiled candy portion and a rapidly disintegrating melt tablet portion.

### Method

The slowly disintegrating hard boiled candy portion is prepared according to Example 6.

To prepare the melt tablet portion with composition as per below Table E1a part of the hydrogenated soybean oil is first melted. Then the solid components, i e the cocoa powder, mannitol, acesulfame-K, and the flavoring agents, if solid, are added and mixed. A reduction of particle size of the solid components is performed by milling in a roll-refiner. If the solid components have already got the required particle size, e g by milling before the mixing with the oil, roll refining is dispensed with. After treatment in the roll-refiner the mixture is mixed with the rest of the melted fatty components or remelted, if solidified, and mixed with the rest of the melted hydrogenated soybean oil. A mixing of the melt is performed in a suitable mixer. The liquid components, i e the soy lecithin and the flavoring agents (if liquid), are added at this stage. The two portions, hard boiled candy and melt tablet, are then combined by dispensing the melt on top of the cooled and hardened hard boiled candy portion in a suitable mold. The melt is then allowed to solidify by cooling at 8-15 °C for 2 hours. The complete dual portion dosage form is then broken from the mold and suitably packaged.

**Table E1: Components of the melt tablet portion.**

| Ingredients | Percent (w/w) | mg/melt tablet portion |
|---|---|---|
| Hydrogenated soybean oil | 40,0 | 80,0 |
| Cocoa powder | 38,3 | 76,6 |
| Mannitol | 20,0 | 40,0 |
| Acesulfame-K | 0,4 | 0,8 |
| Flavoring agents | 1,0 | 2,0 |
| Soy lecithin | 0,7 | 1,4 |
| TOTAL | 100,0 | 200,0 |

### Comparative Example 8

Preparation of seamless softgel concentric triple portion intra-oral capsules.

**Table F1: Components of the triple portion capsules.**

| Ingredients | Percent in portion (w/w) | mg/capsule |
|---|---|---|
| *Ingredients in Centre Core* | | |
| *Portion:* | | |
| | 2.2 | 2.0 |
| Nicotine free base | 91.8 | 83.5 |
| Medium chain triglycerides | 5.5 | 5.0 |
| Flavors and sweeteners Colloidal silicon dioxide | 0.5 | 0.5 |
| | | |
| *Ingredients of Inner Shell* | | |
| *Portion:* | 58.0 | 24.7 |
| | 38.0 | 16.2 |
| Sucrose fatty acid ester Hydrogenated vegetable oil Sodium carbonate anhydrous | 4.0 | 1.7 |
| | | |
| *Ingredients of Outer Shell* | 77.0 | 6.5 |
| *Portion:* | 18.0 | 1.5 |
| | 3.0 | 0.3 |
| Gelatin | 2.0 | 0.2 |
| Sorbitol | | |
| Flavors and sweeteners | | |
| Glycerin | | |
| *Weight Ratio:* | | |
| *Core*/*Inner shell*/*Outer shell* | 64/30/6 % | |
| *Total Capsule weight:* | | 142.1 mg |

### Method

Seamless softgel capsules are manufactured by formation of droplets consisting of two or more concentric layers with ingredients as per above Table F1. The droplets are formed by feeding different liquids through concentric nozzles. The outermost nozzle feeds a hydrophilic solution consisting of gelatin and additives e g plasticizers. The one or more inner nozzles feed a lipophilic liquid (e g oils, triglycerids) wherein one or more active substances may be dispersed. The lipophilic centre and hydrophilic perimeter of the formed droplets ensure a good phase separation between shell and core contents. The formed capsules are then subjected to sequential processing steps such as cooling, drying, washing and selection of size and shape.

### Example 9.

Preparation of a Sugar-free Chewing Sweet

A chewy dual portion dual portion formulation where the rapidly disintegrating portion contains 1 mg nicotine (NRC) together with menthol flavor and the slowly disintegrating portion contains 2 mg nicotine (NRC) with a lemon flavor may be prepared by essentially using the method described in US 6,372,271B1. Optionally a stabilising layer may be added to the soft caramel mixture.

### Preparation of the Soft Caramel Mixture For the Centre

### Method

ISOMALT.RTM. (Type M), maltitol syrup and water are heated at 125-135°C, preferably 131°C, in a boiler. Add the gelatine solution. Add vegetable fat, emulsifier, citric acid, ISOMALT.RTM. (Type PF) in the given sequence, while mixing at high speed for 2 to 3 minutes until an homogenous mixture is obtained. Add fruit flavouring, mix, and empty the boiler. Homogenise using a suitable homogeniser. Cool the mixture to 42 to 48°C Pulling time for the mixture for the centre: 1 to 15 minutes, preferably 8 minutes. The preparation of the soft caramel mix can be carried out in a batch cooker or continuous cooking equipment. Pulling of the mixture is carried out with standard pulling machines or continuous pulling machines or, in the case of aeration, with standard aerators.

*Forming of the Mixture:* Processing of the mixture is carried out in the normal way, in which the forming of the fillings is performed by an embossing machine. The surface temperature of the rope before the stamping operation is not greater than 35°C After stamping, the fillings pass through a cooling tunnel. Afterwards, the temperature is 10 to 30°C, preferably 25°C

*Pregumming*: Immediately after leaving the cooling tunnel, the fillings are collected in containers and pregummed. For this purpose, a 50% Quick Coat solution (gum arabic, Wolff & Olsen, Hamburg) with 10% titanium dioxide is prepared, which is applied in one amount to the fillings so that the filings are well-moistened, then the applied solution is sprinkled with Quick Coat powder until the fillings are dry. This process is repeated up to two or three times so that the fillings are stabilized against changes in volume and do not stick together.

**Table G1: Composition of the rapidly disintegrating portion**

| Ingredients | Percent (w/w) | mg/portion |
|---|---|---|
| Nicotine resin complex (20% nicotine) | 0.75 | 15* |
| ISOMALT .RTM. (Type M) | 24.20 | 484 |
| Maltitol syrup (75% TS) | 49.70 | 994 |
| ISOMALT .RTM. (Type PF) | 8.40 | 168 |
| Vegetable fat (34-36.degree. Sp) | 5.80 | 116 |
| Water | 5.00 | 100 |
| Gelatin 120 Bloom (40%) | 3.55 | 71 |
| Trometamol) | 0.5 | 10 |
| Sodium Carbonate | 0.25 | 5 |
| Emulsier | 0.75 | 15 |
| Citric acid (monohydrate) | 0.70 | 14 |
| Lemon flavor | 0.40 | 8 |
| TOTAL | 100.0 | 2000 |

| | | |
|---|---|---|
| * Equivalent to a 3.0 mg dose of nicotine. | | |

*Sweet Coating:* Preparation of the Solution. ISOMALT.RTM. (Type M) is mixed in warm water and heated to 70 to 80°C until the solution is free of crystals. Preparation of the Suspension: The solution prepared as previously described is cooled to 60°C Aspartame, acesulfame K, gum arabic solution, TiO₂ and ISOMALT.RTM. (Type PF) are added and stirred until a homogeneous mixture is obtained. The temperature of the suspension is maintained at 60°C during the process.

**Table G2: Components of the slowly disintegrating portion.**

| Ingredients | Percent (w/w) | mg/portion |
|---|---|---|
| Nicotine bitartrate dihydrate | 0.88 | 3.08* |
| ISOMALT .RTM. (Type M) | 40.23 | 140.80 |
| Water | 29.00 | 101.5 |
| ISOMALT .RTM. (Type PF) | 22.15 | 77.53 |
| Gum arabic (solution 1:1) | 4.10 | 14.35 |
| Trometamol) | 2.14 | 7.49 |
| TiO₂ | 1.00 | 3.5 |
| Menthol | 0.4 | 1.4 |
| Acesulfame K | 0.05 | 0.175 |
| Aspartame | 0.05 | 0.175 |
| TOTAL | 100.0 | 350 |

| | | |
|---|---|---|
| * Equivalent to a 1.0 mg dose of nicotine. | | |

A stabilizing layer consisting of a soft caramel mixture may also be included.

### Example 10.

Preparation of a tablet containing 2mg nicotine and 10x10⁶ cfu Lactobacillus reuteri ATCC PTA-5289

Preparation of a dual portion tablet where the rapidly disintegrating portion contains Lactobacillus reuteri for improved oral health together with fruit flavor and the slowly disintegrating portion contains 2.0 mg nicotine (NRC) with a mint flavor

### Method

The same method as in Example 1 is used.

**Table H1: Components of the rapidly disintegrating tablet portion.**

| Ingredients | mg or amount / portion |
|---|---|
| Lactobacillus reuteri ATCC PTA-5289 | 10x10⁶ cfu |
| Crospovidone | 3 |
| Microcrystalline Cellulose (Avicel PH100) 100101) | 20 |
| Dextrose Monohydrate | 360 |
| Fruit flavor | 1 |
| Coloring agent | 0.04 |
| Magnesium Stearate | 3 |

**Table H2: Components of the slowly disintegrating portion.**

| Ingredients | Percent (w/w) | mg/portion |
|---|---|---|
| Nicotine resin complex (20% nicotine) | 1 | 10* |
| Sorbitol | 95.5 | 955 |
| Trometamol | 1.0 | 10 |
| Sodium Carbonate | 0.5 | 5 |
| Mint flavor | 1 | 10 |
| Magnesium Stearate | 1 | 10 |
| TOTAL | 100.0 | 1000.0 |

| | | |
|---|---|---|
| * Equivalent to a 2.0 mg dose of nicotine. | | |

### Example 11.

Preparation of a tablet containing Terbutaline sulfate 5mg and Loratadine 10mg

**Table A1 : Components of the rapidly disintegrating tablet portion.**

| Ingredients | Percent (w/w) | Mg/portion |
|---|---|---|
| Terbutaline sulfate | 1.67 | 5 |
| Crospovidone | 3.33 | 10 |
| Mannitol | 93.15 | 279.46 |
| Menthol | 0.33 | 1 |
| Sweetner | 0.50 | 1.5 |
| Coloring agent | 0.01 | 0.04 |
| Magnesium Stearate | 1.00 | 3 |
| TOTAL | 100.00 | 300 |

**Table A2: Components of the slowly disintegrating portion.**

| Ingredients | Percent (w/w) | mg/portion |
|---|---|---|
| Loratadin | 1 | 10 |
| Sorbitol | 97 | 966.5 |
| Citric acid | 0.35 | 3.5 |
| Lemon flavor | 1 | 10 |
| Magnesium Stearate | 1 | 10 |
| TOTAL | 100.0 | 1000.0 |

Preparation of a dual portion tablet where the rapidly disintegrating portion contains terbutaline sulfate as a beta-adrenergic agonist bronchodilator together with menthol and the slowly disintegrating portion contains Loratadine with a lemon flavor.

### Method

The same method as in Example 1 is used.

Also many other embodiments than those presented in the captioned examples are encompassed by the present invention.

One such other embodiment is e g a chewy preparation with a hard coating. The centre of such a preparation may be a soft caramel mixture comprising nicotine resin complex, isomalt, maltitol syrup, vegetable fat, gelatine, emulsifier, buffer and flavour. This soft centre may be manufactured using conventional technology. The centre is subsequently hard coated with a coating solution comprising nicotine bitartrate dihydrate, isomalt, gum arabic, buffer, sweetener, and flavour. It should be noted that one form of nicotine is used in the centre and another form of nicotine is used in the coating. Optionally a a thin stabilizing layer, consisting of a soft caramel mixture, may placed between the soft centre and the hard coating.

## Claims

1. A multi portion intra-oral dosage form comprising a component for treating tobacco dependence, which is nicotine and/or metabolites thereof, selected from cotinine, nicotine N'-oxide, nornicotine and (S)-nicotine-N-β-glucuronide and mixtures, isomers, salts and complexes thereof in any form,
wherein at least one portion of the multi portion intra-oral dosage form is rapidly disintegrating and at least one portion is slowly disintegrating, whereby the disintegration time for the slowest disintegrating portion is at least two times longer than for the most rapidly disintegrating portion, and where each portion comprises at least one item selected from the following items:
a pharmaceutically active component, a nicotine mimicking component, a pH-buffering component, a pH-regulating component, a flavor, a barrier component, a color component, an adhesive component, a taste masking agent, a tooth whitening agent, a breath freshening agent, an oral health promoting agent, an anti-caries agent, and an anti-inflammatory agent; and
wherein the at least one rapidly disintegrating portion further comprises a water-swellable excipient selected from sodium starch glycolate, crospovidone, croscarmellose, microcrystalline cellulose, starches, hydroxypropyl cellulose, and alginic acid.

2. A multi portion intra-oral dosage form according to claim 1, where a pharmaceutically active component is a component for treating tobacco dependence.

3. A multi portion intra-oral dosage form according to claim 1 or 2 not being a chewing gum or triturate.

4. A multi portion intra-oral dosage form according to any of the preceding claims,
comprising a buffer and/or a pH-adjusting agent, which upon administration to a subject transiently elevates the pH of the saliva of the subject by 0.2 - 3.5 pH units, preferably by 0.5-2.0 pH units.

5. A multi portion intra-oral dosage form according to any of claims 2 - 4, where at least one of the rapidly and the slowly disintegrating portions comprises a component for creating a noticeable organoleptic sensation; wherein preferably the organoleptic sensation/s is/are in the form of one or more of perception of or a change in the perception of flavor, cooling, burning, warming, heating, crunching, tingling, bubbling, foaming, effervescing, mouth watering, physical form, stickiness, and texture, e g hardness, softness, roughness and engravings; wherein preferably the organoleptic sensation/s is/are such that it/they facilitate/s for a subject using the dosage form to differentiate between different portions thereof; and wherein preferably the organoleptic sensation is a perception of flavor or a change in perception of flavor.

6. A multi portion intra-oral dosage form according to claim 5, where an organoleptic sensation is delivered from a portion as a signal to inform a subject using the dosage form that a certain fraction of, e g more than three quarters of, all of or nearly all of, a pharmaceutically active ingredient initially being present in said portion has been released there from.

7. A multi portion intra-oral dosage form according to claim 5 or claim 6, where an organoleptic sensation is delivered from a portion as a signal to inform a subject using the dosage form that a pharmaceutically active ingredient being present in said portion has started to be released there from.

8. A multi portion intra-oral dosage form according to any preceding claim, where
**I.** the rapidly and the slowly disintegrating portions comprise the same pharmaceutically active agent, or
**II.** the rapidly and the slowly disintegrating portions comprise different pharmaceutically active agents, or
**III.** the rapidly and the slowly disintegrating portions, regardless of their respective disintegration time, have the same or different pharmaceutically active agents, and/or
**IV.** the dosage form provides for including non-compatible ingredients, such as flavor components, buffers and pharmaceutically active agents that are not compatible, by formulating such ingredients in separate portions

9. A multi portion intra-oral dosage form according to any preceding claim, where the disintegration time for the most slowly disintegrating portion is 3 - 10 times longer, preferably 3 - 5 times longer, than for the most rapidly disintegrating portion.

10. A multi portion intra-oral dosage form according to any preceding claim, where the rapidly disintegrating portion(s) comprise(s) an effervescent.

11. A multi portion intra-oral dosage form according to any preceding claim, where the at least one rapidly disintegrating portion at least partly covers the at least one slowly disintegrating portion.

12. A multi portion intra-oral dosage form according to any preceding claim, where the slowly disintegrating portion(s) cover(s) at least partly covers the surface of the rapidly disintegrating portion.

13. A multi portion intra-oral dosage form according to any preceding claim, being a lozenge, a tablet, an oral film, a sublingual tablet, a troche, a lolly pop, a hard boiled candy, a chocolate lens, a micro bead, a jelly, a jelly bean, a wine gum, a semi solid, a center filled dosage form or a combination thereof

14. A multi portion intra-oral dosage form according to any preceding claim, wherein each disintegrating portion is composed of two or more sub portions, each sub portion comprising a pharmaceutically active agent, whereby the pharmaceutically active agent in at least one of said sub portions is coated.

15. A multi portion intra-oral dosage form according to any preceding claim comprising a component for treating tobacco dependence, which is selected from the group consisting of a nicotine salt, the free base form of nicotine, a nicotine derivative, such as a nicotine cation exchanger, a nicotine inclusion complex or nicotine in any non-covalent binding, nicotine bound to zeolites, nicotine bound to cellulose or starch micro-spheres, a nicotine pro-drug, and/or mixtures thereof; wherein the nicotine inclusion complex is preferably a cyclodextrin complex, where the cyclodextrin used is chosen among α-, β- and γ-cyclodextrin, hydroxypropyl derivatives of α-, P- and γ-cyclodextrin, sulfoalkylether cyclodextrins such as sulfobutylether β-cyclodextrin, alkylated cyclodextrins such as randomly methylated β-cyclodextrin, and branched cyclodextrins such as glucosyl- and maltosyl-β-cyclodextrin; and wherein the nicotine cation exchanger is preferably a polyacrylate cation exchanger; and wherein the nicotine salt is preferably, mono-tartrate, hydrogen tartrate, citrate, malate and/or hydrochloride salt; wherein said nicotine and/or metabolites thereof is present in an amount of 0.05 - 12 mg, preferably in an amount of 0.1 - 6 mg, more preferably in an amount of 1 - 6 mg, and most preferably in an amount of 2 - 5 mg calculated as the free base form of nicotine per unit dose..

16. A multi portion intra-oral dosage form according to any preceding claim, comprising a component for treating tobacco dependence being selected from one or more of varenicline, bupropion, nortriptyline, doxepin, fluoxetine, imipramine, moclobemide, and/or cytisine.

17. A multi-portion intra-oral dosage form according to any one of claims 1 - 16 for use in obtaining a quick and/or sustained and/or complete reduction of the urge to smoke or use of tobacco.

18. A multi portion intra-oral dosage form according to any one of claims 1 - 16, wherein at least one rapidly disintegrating portion comprises a compressible excipient selected from isomalt, dextrose monohydrate, maltodextrin, lactose monohydrate, dextrin, mannitol, lactitol, sorbitol, xylitol, erythritol, sucrose, and lactose, and mixtures or derivatives thereof; wherein preferably the rapidly disintegrating portion comprises at least 40 % by weight of a compressible excipient selected from isomalt, dextrose monohydrate, maltodextrin, lactose monohydrate, dextrin, mannitol, lactitol, sorbitol, xylitol, erythritol, sucrose, and lactose, and mixtures thereof; wherein preferably the compressible excipient is in the form of particles with an average particle diameter of about 50 to about 400 microns.

19. A multi portion intra-oral dosage form according to claim 18, wherein the weight ratio of the compressible excipient to the water-swellable excipient is from about 10:1 to about 500:1.

20. A multi portion intra-oral dosage form according to any of claims 1 - 16, wherein at least one rapidly disintegrating portion further comprises a pH-buffer component and/or a pH adjusting component selected from a carbonate including bicarbonate or sesquicarbonate, glycinate, phosphate, glycerophosphate or citrate of an alkali metal, such as potassium or sodium, or ammonium, including trisodium phosphate, disodium hydrogen phosphate; tripotassium phosphate, dipotassium hydrogen phosphate, and calcium hydroxide, sodium glycinate, trometamol or an amino acid; and mixtures thereof.

21. A multi portion intra-oral dosage form according to any of claims 1 - 16 or 18 - 20, wherein the rapidly disintegrating portion or portions has/have a hardness of less than about 15 kp/cm², and the slowly disintegrating portion or portions has/have a hardness of greater than about 15 kp/ cm².

22. A multi portion intra-oral dosage form according to any of claims 1 - 16 or 18 - 21, wherein the slowly disintegrating portion or portions comprise(s) a excipient selected from, but not limited to, the group consisting of isomalt, sucrose, dextrose, dextrose monohydrate, corn syrup, lactitol, lycasin, mannitol, sorbitol, erythritol, xylitol, starches, gelatinized starches, maltodextrin, lactose, lactose monohydrate, dextrin, and mixtures and/or derivatives thereof

23. A multi portion intra-oral dosage form according to any of claims 1 - 16 or 18 -22, wherein the slowly disintegrating portion or portions comprise(s) at least 50%, by weight, of a sugar selected from isomalt, sucrose, dextrose, corn syrup, lactitol, and lycasin, and mixtures and/or derivatives thereof; wherein preferably the rapidly disintegrating portion or portions further comprise(s) an effervescent couple comprising a member selected from the group consisting of sodium bicarbonate, potassium bicarbonate, calcium carbonate, magnesium carbonate, and sodium carbonate and one member selected from the group consisting of citric acid, malic acid, fumaric acid, tartaric acid, and alginic acid.

24. A multi portion intra-oral dosage form according to any of claims 1 - 16 or 18-23, wherein the pharmaceutically active agent is in the form of particles that are further coated with a taste-masking polymer and wherein the average particle diameter of the particles is from about 50 microns to about 1000 microns.

25. A multi portion intra-oral dosage form according to any of claims 1 - 16 or 18 -24, wherein at least one of the slowly disintegrating portions comprises a plurality of openings exposing the surface area of the this/these portion/s, and substantially covers the surface area of at least of the rapidly disintegrating portions, whereby said slowly disintegrating portion/s further comprise/s a plurality of indentations that, upon contact with the fluids in the oral cavity, are adapted to dissolve and expose the surface area of the rapidly disintegrating portion/s.

26. A multi portion intra-oral dosage form according to any of claims 1 - 16 or 18 - 25, wherein a pharmaceutically active agent within a rapidly disintegrating portion is selected from the group consisting of zinc, chlorhexidine, L. reuteri, nystatin, amphotericin, miconazole, phenylephrine, dextromethorphan, pseudoephedrine, acetaminophen, ibuprofen, ketoprofen, loperamide, famotidine, calcium carbonate, simethicone, pseudoephedrine, chlorpheniramine, methocarbomal, chlophedianol, ascorbic acid, menthol, pectin, dyclonine, benzocaine, and menthol, and pharmaceutically acceptable salts and derivatives thereof.

27. A multi portion intra-oral dosage form according to any of claims 1 - 16 or 18 - 26, wherein the face of at least one portion has a convex shape and the face of an adjoining portion has a concave shape.

28. A multi portion intra-oral dosage form according to any of claims 1 - 16 or 18 - 27 having geometric similarities to a sphere, an open or closed oblong object, a sandwich, a hamburger or a torus.

29. A multi portion intra-oral dosage form according to any of claims 1 - 16 or 18 - 28 having inter-portions layer/s comprising an edible adhesive-like material; wherein preferably the edible adhesive-like material comprises an ingredient selected from the group consisting of polyethylene glycol, polyethylene oxide, polycaprolactone, carnauba wax, microcrystalline wax, oppanol, shellac wax and beeswax.

30. A multi portion intra-oral dosage form according to any of claims 1 - 16 or 18 - 29, wherein at least one rapidly disintegrating portion comprises at least one pharmaceutically active agent selected from the group of phenylephrine, dextromethorphan, chlorpheniramine, chlophedianol, and pseudoephedrine, and wherein at least one slowly disintegrating portion comprises at least one pharmaceutically active agent selected from the group of menthol, nicotine, dyclonine, pectin, benzocaine, thymol, methyl salicylate and eucalyptol.

31. A multi portion intra-oral dosage form according to any to any of claims 1 - 16 or 18 - 30 where at least one rapidly disintegrating portion is substantially free from nicotine, whereby substantially free is defined as containing 0.05 mg per unit dose or less.

32. A multi portion intra-oral dosage form according to any of claims 1 - 16 or 18 - 31 where at least one rapidly disintegrating portion is a compressed portion and where at least one slowly disintegrating portion has a matrix that is a hard candy glass.

33. A multi portion inter-oral dosage form according to any preceding claim, where the slowly or the rapidly disintegrating portion(s) have indentation(s) and/or holes filled by the other disintegrating portion(s).

34. A multi portion inter-oral dosage form according to any of claims 1 - 16 or 18 - 33 comprising nicotine and/or metabolites thereof, such as cotinine, nicotine N'-oxide, nornicotine, (S)-nicotine-N-β-glucuronide and mixtures, isomers, salts and complexes thereof in any form for use in therapy wherein the therapy is treatment of a disease selected from the group consisting of tobacco or nicotine dependence, Alzheimer's disease, Crohn's disease, Parkinson's disease, Tourette's syndrome, ulcerous colitis.

35. A multi portion inter-oral dosage form according to any preceding claims, **characterized in that** the one or more pharmaceutically active agent(s) is/are chosen from the antiinflammatory agents diclofenac, ketorolac, indometacin, tornoxicam, piroxicam, tenoxicam, ketoprofen, celecoxib and roficoxib; the muscle relaxants orphenadrine and baclofen; the drugs affecting bone mineralization alendronic acid and risedronic acid; the analgesics propoxyphene, buprenorfin, ketobenidon, hydromorphone, tramadol, morphine, and tapentadol; the antimigraine preparations: dihydroergotamine, ergotamine, eletriptan, naratriptan, rizatriptan, sumatriptan and zolmitriptan; the anti-Parkinson drugs pramipexole, ropinirole and selegiline; the anxiolytics alprazolam, diazepam, lorazepam and oxazepam; the hypnotics flunitrazepam, midazolam, nitrazepam, triazolam, zaleplone, zopiclone, zolpiderm, clometiazole and propiomazine; the psychostimulant caffeine; the drugs against substance dependence bupropione, lobeline, naltrexone and methadone; the gastric ulcer remedy famotidine; the antispasmodic hyoscyamine; the antiemetics metoclopramide, ondansetron, scopolamine, hyoscine, perfenazine, procloperazine and haloperidol; the antidiabetic agent rosiglitazone; the cardiovascular agents etilefrin, glyceryl trinitrate, isosorbide dinitrate and isosorbide mononitrate; the antihypertensive agent hydralazine; the diuretics furosemide and amiloride; the beta-receptor blocking agents propranolol and timolol; the calcium channel blocker amlodipine; the ACE-inhibitors kaptopril, lisinopril and fosinopril; the serum lipid reducing agent simvastatin; the antipsoriatic acitretin; the antiasthmatic terbutaline; the antitussives codeine and noscapine, and the antihistamines clemastine, chlorpheniramine, cyproheptadine, loratadine and acrivastine; the antidepressant and anti-sexual dysfunction drug dapoxetine; the anti-sexual dysfunction drugs sildenafil (Viagra), tadalafil, vardenafil, cabergoline and pramipexole, the antiepileptic topiramate, and
the oral and/or gastrointestinal and/or general health promoting agent Lactobacillus reuteri.

36. A multi portion inter-oral dosage form according to claim 35, **characterized in that** it comprises nicotine and Lactobacillus reuteri.

37. A multi portion inter-oral dosage form according to claim 35, **characterized in that** it comprises terbutaline and loratadine.

## Patentansprüche

1. Mehrteilige intraorale Darreichungsform, umfassend eine Komponente zum Behandeln von Tabakabhängigkeit, bei der es sich um Nicotin und/oder Metaboliten davon ausgewählt aus Cotinin, Nicotin-N'-oxid, Nornicotin und (S)-Nicotin-N-β-glucuronid und Gemischen, Isomeren, Salzen und Komplexen davon in jeder Form handelt,
wobei wenigstens ein Teil der mehrteiligen intraoralen Darreichungsform schnell zerfallend ist und wenigstens ein Teil langsam zerfallend ist, wobei die Zerfallszeit für den am langsamsten zerfallenden Teil wenigstens zweimal länger als für den am schnellsten zerfallenden Teil ist und wobei jeder Teil wenigstens ein Element ausgewählt aus den folgenden Elementen umfasst:
eine pharmazeutisch wirksame Komponente, eine Nicotin-nachahmende Komponente, eine pH-Wertpuffernde Komponente, eine pH-Wertregulierende Komponente, einen Aromastoff, eine Barrierekomponente, eine Farbkomponente, eine Haftkomponente, eine geschmacksmaskierende Komponente, eine zahnweißende Komponente, eine atemerfrischende Komponente, eine die Mundgesundheit fördernde Komponente, ein Antikariesmittel und ein entzündungshemmendes Mittel; und
wobei der wenigstens eine schnell zerfallende Teil ferner einen wasserquellbaren Hilfsstoff ausgewählt aus Natriumstärkeglycolat, Crospovidon, Croscarmellose, mikrokristalliner Cellulose, Stärken, Hydroxypropylcellulose und Alginsäure umfasst.

2. Mehrteilige intraorale Darreichungsform gemäß Anspruch 1, wobei eine pharmazeutisch wirksame Komponente eine Komponente zum Behandeln von Tabakabhängigkeit ist.

3. Mehrteilige intraorale Darreichungsform gemäß Anspruch 1 oder 2, die kein Kaugummi oder Triturat ist.

4. Mehrteilige intraorale Darreichungsform gemäß einem der vorstehenden Ansprüche, umfassend einen Puffer und/oder ein pH-Wert-einstellendes Mittel, das bei Verabreichung an ein Subjekt den pH-Wert der Speichelflüssigkeit des Subjekts vorübergehend um 0,2-3,5 pH-Wert-Einheiten anhebt, vorzugsweise um 0,5-2,0 pH-Wert-Einheiten.

5. Mehrteilige intraorale Darreichungsform gemäß einem der Ansprüche 2-4, wobei wenigstens einer der schnell und der langsam zerfallenden Teile eine Komponente zum Erzeugen eines wahrnehmbaren organoleptischen Sinneseindrucks umfasst; wobei der organoleptische Sinneseindruck bzw. die organoleptischen Sinneseindrücke vorzugsweise in der Form von einem oder mehreren von einer Wahrnehmung von oder einer Veränderung der Wahrnehmung von Aroma, Kühlen, Brennen, Wärmen, Erhitzen, Knuspern, Kitzeln, Sprudeln, Schäumen, Brausen, wässrigem Mund, Aggregatform, Klebrigkeit und Textur, beispielsweise Härte, Weichheit, Rauigkeit und Gravuren, ist/sind; wobei der organoleptische Sinneseindruck bzw. die organoleptischen Sinneseindrücke vorzugsweise so ist/sind, dass sie für ein Subjekt, das die Darreichungsform verwendet, Unterscheiden zwischen verschiedenen Teilen davon ermöglicht/ermöglichen; und wobei der organoleptische Sinneseindruck vorzugsweise eine Wahrnehmung von Aroma oder eine Veränderung der Wahrnehmung von Aroma ist.

6. Mehrteilige intraorale Darreichungsform gemäß Anspruch 5, wobei ein organoleptischer Sinneseindruck von einem Teil als Signal geliefert wird, um ein Subjekt, das die Darreichungsform verwendet, darüber zu informieren, dass ein bestimmter Anteil, beispielsweise mehr als drei Viertel oder beinahe alles von einem pharmazeutischen Wirkstoff, der anfangs in dem Teil vorhanden war, daraus freigesetzt worden ist.

7. Mehrteilige intraorale Darreichungsform gemäß Anspruch 5 oder Anspruch 6, wobei ein organoleptischer Sinneseindruck von einem Teil als Signal geliefert wird, um ein Subjekt, das die Darreichungsform verwendet, darüber zu informieren, dass ein pharmazeutischer Wirkstoff, der in dem Teil vorhanden ist, begonnen hat, daraus freigesetzt zu werden.

8. Mehrteilige intraorale Darreichungsform gemäß einem der vorstehenden Ansprüche, wobei
I. die schnell und die langsam zerfallenden Teile den gleichen pharmazeutischen Wirkstoff umfassen oder
II. die schnell und die langsam zerfallenden Teile verschiedene pharmazeutische Wirkstoffe umfassen oder
III. die schnell und die langsam zerfallenden Teile unabhängig von ihrer jeweiligen Zerfallszeit den gleichen oder verschiedene pharmazeutische Wirkstoffe aufweisen und/oder
IV. die Darreichungsform das Einschließen unvereinbarer Inhaltsstoffe, wie z. B. Aromakomponenten, Puffer und pharmazeutische Wirkstoffe, die nicht vereinbar sind, durch Formulieren der Inhaltsstoffe in getrennten Teilen ermöglicht.

9. Mehrteilige intraorale Darreichungsform gemäß einem der vorstehenden Ansprüche, wobei die Zerfallszeit für den am langsamsten zerfallenden Teil 3-10-mal länger, vorzugsweise 3-5-mal länger, als für den am schnellsten zerfallenden Teil ist.

10. Mehrteilige intraorale Darreichungsform gemäß einem der vorstehenden Ansprüche, wobei der/die schnell zerfallende(n) Teil(e) ein Brausemittel umfasst/umfassen.

11. Mehrteilige intraorale Darreichungsform gemäß einem der vorstehenden Ansprüche, wobei der wenigstens eine schnell zerfallende Teil den wenigstens einen langsam zerfallenden Teil wenigstens teilweise bedeckt.

12. Mehrteilige intraorale Darreichungsform gemäß einem der vorstehenden Ansprüche, wobei der/die langsam zerfallende(n) Teil(e) die Oberfläche des schnell zerfallenden Teils wenigstens teilweise bedeckt/bedecken.

13. Mehrteilige intraorale Darreichungsform gemäß einem der vorstehenden Ansprüche, die eine Lutschtablette, eine Tablette, ein Oralfilm, eine Sublingualtablette, eine Pastille, ein Lollipop, ein Bonbon, eine Schokoladelinse, ein Mikrokügelchen, ein Gelee, ein Geleebonbon, ein Weingummi, ein halbfester Stoff, eine in der Mitte gefüllte Darreichungsform oder eine Kombination davon ist.

14. Mehrteilige intraorale Darreichungsform gemäß einem der vorstehenden Ansprüche, wobei jeder zerfallende Teil aus zwei oder mehr Unterteilen besteht, wobei jeder Unterteil einen pharmazeutischen Wirkstoff umfasst, wobei der pharmazeutische Wirkstoff in wenigstens einem der Unterteile beschichtet ist.

15. Mehrteilige intraorale Darreichungsform gemäß einem der vorstehenden Ansprüche, umfassend eine Komponente zum Behandeln von Tabakabhängigkeit, die ausgewählt ist aus der Gruppe bestehend aus einem Nicotinsalz, der freien Baseform von Nicotin, einem Nicotinderivat, wie z. B. einem Nicotinkationenaustauscher, einem Nicotin-Einschlusskomplex oder Nicotin in einer beliebigen nichtkovalenten Bindung, an Zeolithe gebundenem Nicotin, an Cellulose oder Stärke-Mikrokügelchen gebundenem Nicotin, einem Nicotin-Prodrug und/oder Gemischen davon; wobei der Nicotin-Einschlusskomplex vorzugsweise ein Cyclodextrinkomplex ist, wobei das verwendete Cyclodextrin ausgewählt ist aus α-, β- und γ-Cyclodextrin, Hydroxypropylderivaten von α-, β-und γ-Cyclodextrin, Sulfoalkylether-Cyclodextrinen, wie z. B. Sulfobutylether-β-cyclodextrin, alkylierten Cyclodextrinen, wie z. B. statistisch methyliertem β-Cyclodextrin, und verzweigten Cyclodextrinen, wie z. B. Glucosyl- und Maltosyl-β-cyclodextrin; und wobei der NicotinKationenaustauscher vorzugsweise ein Polyacrylat-Kationenaustauscher ist; und wobei das Nicotinsalz vorzugsweise Monotartrat-, Hydrogentartrat-, Citrat-, Malat- und/oder Hydrochloridsalz ist; wobei das Nicotin und/oder die Metaboliten davon in einer Menge von 0,05-12 mg, vorzugsweise in einer Menge von 0,1-6 mg, bevorzugter in einer Menge von 1-6 mg und höchst bevorzugt in einer Menge von 2-5 mg, berechnet als die freie Base von Nicotin, pro Einheitsdosis vorhanden ist.

16. Mehrteilige intraorale Darreichungsform gemäß einem der vorstehenden Ansprüche, umfassend eine Komponente zum Behandeln von Tabakabhängigkeit ausgewählt aus einem oder mehreren von Vareniclin, Bupropion, Nortriptylin, Doxepin, Fluoxetin, Imipramin, Moclobemid und/oder Cytisin.

17. Mehrteilige intraorale Darreichungsform gemäß einem der Ansprüche 1-16 für die Verwendung zum Erhalten einer schnellen und/oder anhaltenden und/oder vollständigen Verringerung des Drangs zum Rauchen oder Verwenden von Tabak.

18. Mehrteilige intraorale Darreichungsform gemäß einem der Ansprüche 1-16, wobei wenigstens ein schnell zerfallender Teil einen komprimierbaren Hilfsstoff ausgewählt aus Isomalt, Dextrosemonohydrat, Maltodextrin, Lactosemonohydrat, Dextrin, Mannit, Lactit, Sorbit, Xylit, Erythrit, Saccharose und Lactose und Gemischen davon umfasst; wobei der schnell zerfallende Teil vorzugsweise wenigstens 40 Gew.-% an einem komprimierbaren Hilfsstoff ausgewählt aus Isomalt, Dextrosemonohydrat, Maltodextrin, Lactosemonohydrat, Dextrin, Mannit, Lactit, Sorbit, Xylit, Erythrit, Saccharose und Lactose und Gemischen davon umfasst; wobei der komprimierbare Hilfsstoff vorzugsweise in der Form von Partikeln mit einem mittleren Partikeldurchmesser von etwa 50 bis etwa 400 Mikrometer vorliegt.

19. Mehrteilige intraorale Darreichungsform gemäß Anspruch 18, wobei das Gewichtsverhältnis des komprimierbaren Hilfsstoffs zu dem wasserquellbaren Hilfsstoff von etwa 10:1 bis etwa 500:1 beträgt.

20. Mehrteilige intraorale Darreichungsform gemäß einem der Ansprüche 1-16, wobei wenigstens ein schnell zerfallender Teil ferner eine pH-Wert-Pufferkomponente und/oder eine pH-Werteinstellende Komponente umfasst, ausgewählt aus einem Carbonat, einschließlich Bicarbonat oder Sesquicarbonat, Glycinat, Phosphat, Glycerophosphat oder Citrat eines Alkalimetalls, wie z. B. Kalium oder Natrium, oder von Ammonium, einschließlich Trinatriumphosphat, Dinatriumhydrogenphosphat, Trikaliumphosphat, Dikaliumhydrogenphosphat, und Calciumhydroxid, Natriumglycinat, Trometamol oder einer Aminosäure; und Gemischen davon.

21. Mehrteilige intraorale Darreichungsform gemäß einem der Ansprüche 1-16 oder 18-20, wobei der/die schnell zerfallende(n) Teil oder Teile eine Härte von weniger als etwa 15 kp/cm² aufweist/aufweisen und der/die langsam zerfallende(n) Teil oder Teile eine Härte von höher als etwa 15 kp/cm² aufweist/aufweisen.

22. Mehrteilige intraorale Darreichungsform gemäß einem der Ansprüche 1-16 oder 18-21, wobei der/die langsam zerfallende(n) Teil oder Teile einen Hilfsstoff ausgewählt aus, aber nicht darauf beschränkt, der Gruppe bestehend aus Isomalt, Saccharose, Dextrose, Dextrosemonohydrat, Maissirup, Lactit, Lycasin, Mannit, Sorbit, Erythrit, Xylit, Stärken, Quellstärken, Maltodextrin, Lactose, Lactosemonohydrat, Dextrin und Gemischen und/oder Derivaten davon umfasst/umfassen.

23. Mehrteilige intraorale Darreichungsform gemäß einem der Ansprüche 1-16 oder 18-22, wobei der/die langsam zerfallende(n) Teil oder Teile wenigstens 50 Gew.-% an einem Zucker ausgewählt aus Isomalt, Saccharose, Dextrose, Maissirup, Lactit und Lycasin und Gemischen und/oder Derivaten davon umfasst/umfassen; und wobei der schnell zerfallende(n) Teil oder Teile vorzugsweise ferner ein Brausepaar umfassend ein Element ausgewählt aus der Gruppe bestehend aus Natriumbicarbonat, Kaliumbicarbonat, Calciumcarbonat, Magnesiumcarbonat und Natriumcarbonat und ein Element ausgewählt aus der Gruppe bestehend aus Citronensäure, Apfelsäure, Fumarsäure, Weinsäure und Alginsäure umfasst/umfassen.

24. Mehrteilige intraorale Darreichungsform gemäß einem der Ansprüche 1-16 oder 18-23, wobei der pharmazeutische Wirkstoff in der Form von Partikeln vorliegt, die ferner mit einem geschmacksmaskierenden Polymer beschichtet sind und wobei der mittlere Partikeldurchmesser der Partikel von etwa 50 Mikrometer bis etwa 1000 Mikrometer beträgt.

25. Mehrteilige intraorale Darreichungsform gemäß einem der Ansprüche 1-16 oder 18-24, wobei wenigstens einer der langsam zerfallenden Teile eine Vielzahl von Öffnungen umfasst, die die Oberfläche dieses Teils/dieser Teile freilegen, und im Wesentlichen die Oberfläche wenigstens eines der schnell zerfallenden Teile bedeckt, wobei der/die langsam zerfallenden Teil(e) ferner eine Vielzahl von Vertiefungen umfasst/umfassen, die dafür ausgelegt sind, sich bei Kontakt mit den Fluiden in der Mundhöhle aufzulösen und die Oberfläche des/der schnell zerfallenden Teils/Teile freizulegen.

26. Mehrteilige intraorale Darreichungsform gemäß einem der Ansprüche 1-16 oder 18-25, wobei ein pharmazeutischer Wirkstoff in einem schnell zerfallenden Teil ausgewählt ist aus der Gruppe bestehend aus Zink, Chlorhexidin, L. reuteri, Nystatin, Amphotericin, Miconazol, Phenylephrin, Dextromethorphan, Pseudoephedrin, Acetaminophen, Ibuprofen, Ketoprofen, Loperamid, Famotidin, Calciumcarbonat, Simethicon, Pseudoephedrin, Chlorpheniramin, Methocarbomal, Chlophedianol, Ascorbinsäure, Menthol, Pectin, Dyclonin, Benzocain und Menthol und pharmazeutisch verträglichen Salzen und Derivaten davon.

27. Mehrteilige intraorale Darreichungsform gemäß einem der Ansprüche 1-16 oder 18-26, wobei die Seite wenigstens eines Teils eine konvexe Form und die Seite eines angrenzenden Teils eine konkave Form aufweist.

28. Mehrteilige intraorale Darreichungsform gemäß einem der Ansprüche 1-16 oder 18-27 mit geometrischen Ähnlichkeiten zu einer Kugel, einem offenen oder geschlossenen langgestreckten Gegenstand, einem Sandwich, einem Hamburger oder einem Torus.

29. Mehrteilige intraorale Darreichungsform gemäß einem der Ansprüche 1-16 oder 18-28 mit einer Schicht/Schichten zwischen Teilen, die ein essbares klebstoffartiges Material umfasst/umfassen; wobei das essbare klebstoffartige Material vorzugsweise einen Inhaltsstoff ausgewählt aus der Gruppe bestehend aus Polyethylenglycol, Polyethylenoxid, Polycaprolacton, Carnaubawachs, mikrokristallinem Wachs, Oppanol, Schellackwachs und Bienenwachs umfasst.

30. Mehrteilige intraorale Darreichungsform gemäß einem der Ansprüche 1-16 oder 18-29, wobei wenigstens ein schnell zerfallender Teil wenigstens einen pharmazeutischen Wirkstoff ausgewählt aus der Gruppe von Phenylephrin, Dextromethorphan, Chlorpheniramin, Chlophedianol und Pseudoephedrin umfasst und wobei wenigstens ein langsam zerfallender Teil wenigstens einen pharmazeutischen Wirkstoff ausgewählt aus der Gruppe von Menthol, Nicotin, Dyclonin, Pectin, Benzocain, Thymol, Methylsalicylat und Eucalyptol umfasst.

31. Mehrteilige intraorale Darreichungsform gemäß einem der Ansprüche 1-16 oder 18-30, wobei wenigstens ein schnell zerfallender Teil im Wesentlichen frei von Nicotin ist, wobei "im Wesentlichen frei von" als 0,05 mg pro Einheitsdosis oder weniger enthaltend definiert ist.

32. Mehrteilige intraorale Darreichungsform gemäß einem der Ansprüche 1-16 oder 18-31, wobei wenigstens ein schnell zerfallender Teil ein komprimierter Teil ist und wobei wenigstens ein langsam zerfallender Teil eine Matrix aufweist, die ein Bonbon-Glas ist.

33. Mehrteilige intraorale Darreichungsform gemäß einem der vorstehenden Ansprüche, wobei der/die langsam oder der/die schnell zerfallende(n) Teil(e) eine Vertiefung/Vertiefungen und/oder Löcher aufweisen, die mit dem/den anderen zerfallenden Teil(en) gefüllt ist/sind.

34. Mehrteilige intraorale Darreichungsform gemäß einem der Ansprüche 1-16 oder 18-33, umfassend Nicotin und/oder Metaboliten davon, wie z. B. Cotinin, Nicotin-N'-oxid, Nornicotin und (S)-Nicotin-N-β-glucuronid und Gemische, Isomere, Salze und Komplexe davon in jeder Form, für die Verwendung bei der Therapie, wobei die Therapie die Behandlung einer Erkrankung ausgewählt aus der Gruppe bestehend aus Tabak- oder Nicotinabhängigkeit, Alzheimer-Erkrankung, Morbus Crohn, Parkinson-Erkrankung, Tourette-Syndrom, Colitis ulcerosa ist.

35. Mehrteilige intraorale Darreichungsform gemäß einem der vorstehenden Ansprüche, **dadurch gekennzeichnet, dass** der eine oder die mehreren pharmazeutische(n) Wirkstoff(e) ausgewählt ist/sind aus den entzündungshemmenden Wirkstoffen Diclofenac, Ketorolac, Indomctacin, Tornoxicam, Piroxicam, Tenoxicam, Ketoprofen, Celecoxib und Roficoxib; den muskelrelaxierenden Mitteln Orphenadrin und Baclofen; den die Knochenmineralisierung beeinflussenden Arzneimitteln Alendronsäure und Risedronsäure; den Analgetika Propoxyphen, Buprenorfin, Ketobenidon, Hydromorphon, Tramadol, Morphin und Tapentadol; den Antimigräne-Präparaten Dihydroergotamin, Ergotamin, Eletriptan, Naratriptan, Rizatriptan, Sumatriptan und Zolmitriptan; den Anti-Parkinson-Arzneimitteln Pramipexol, Ropinirol und Selegilin; den Anxiolytika Alprazolam, Diazepam, Lorazepam und Oxazepam; den Hypnotika Flunitrazepam, Midazolam, Nitrazepam, Triazolam, Zaleplon, Zopiclon, Zolpiderm, Clometiazol und Propiomazin; dem Psychostimulans Coffein; den Arzneimitteln gegen Stoffabhängigkeit Bupropion, Lobelin, Naltrexon und Methadon; dem Magengeschwür-Heilmittel Famotidin; dem Antispasmodikum Hyoscyamin; den Antiemetika Metoclopramid, Ondansetron, Scopolamin, Hyoscin, Perfenazin, Procloperazin und Haloperidol; dem Antidiabetikum Rosiglitazon; den Herz-Kreislauf-Mitteln Etilefrin, Glyceryltrinitrat, Isosorbiddinitrat und Isosorbidmononitrat; dem Mittel gegen Bluthochdruck Hydralazin; den Diuretika Furosemid und Amilorid; den beta-Rezeptor-Blockern Propranolol und Timolol; dem Calciumkanal-Blocker Amlodipin; den ACE-Hemmern Kaptopril, Lisinopril und Fosinopril; dem Serumlipid-senkenden Mittel Simvastatin; dem Antipsoriatikum Acitretin; dem Antiasthmatikum Terbutalin; den Antitussiva Codein und Noscapin; und den Antihistaminika Clemastin, Chlorpheniramin, Cyproheptadin, Loratadin und Acrivastin; dem Antidepressivum und Arzneimittel gegen sexuelle Dysfunktion Dapoxetin; den Arzneimitteln gegen sexuelle Dysfunktion Sildenafil (Viagra), Tadalafil, Vardenafil, Cabergolin und Pramipexol; dem Antiepileptikum Topiramat und dem die orale und/oder Magen-Darm- und/oder Allgemeingesundheit fördernden Mittel Lactobacillus reuteri.

36. Mehrteilige intraorale Darreichungsform gemäß Anspruch 35, **dadurch gekennzeichnet, dass** sie Nicotin und Lactobacillus reuteri umfasst.

37. Mehrteilige intraorale Darreichungsform gemäß Anspruch 35, **dadurch gekennzeichnet, dass** sie Terbutalin und Loratadin umfasst.

## Revendications

1. Forme pharmaceutique intraorale à parties multiples comprenant un composant pour traiter la dépendance au tabac, qui est la nicotine et/ou des métabolites de celle-ci, choisis parmi la cotinine, le N'-oxyde de nicotine, la nornicotine et le (S)-nicotine-N-β-glucuronide et des mélanges, des isomères, des sels et des complexes de ceux-ci sous une forme quelconque,
où au moins une partie de la forme pharmaceutique intraorale à parties multiples se désintègre rapidement et au moins une partie se désintègre lentement, le temps de désintégration pour la partie à désintégration la plus lente est au moins deux fois plus long que pour la partie à désintégration la plus rapide, et où chaque partie comprend au moins un élément choisi parmi les éléments suivants :
un composant pharmaceutiquement actif, un composant mimant la nicotine, un composant tampon de pH, un composant régulateur de pH, un arôme, un composant de barrière, un composant colorant, un composant adhésif, un agent de masquage du goût, un agent de blanchiment des dents, un agent rafraîchisseur de l'haleine, un agent favorisant la santé orale, un agent anti-carie, et un agent anti-inflammatoire ; et où l'au moins une partie à désintégration rapide comprend en outre un excipient gonflable dans l'eau choisi parmi le glycolate d'amidon sodique, la crospovidone, la croscarmellose, la cellulose microcristalline, des amidons, l'hydroxypropylcellulose et l'acide alginique.

2. Forme pharmaceutique intraorale à parties multiples selon la revendication 1, où un composant pharmaceutiquement actif est un composant pour traiter la dépendance au tabac.

3. Forme pharmaceutique intraorale à parties multiples selon la revendication 1 ou 2 n'étant pas une gomme à mâcher ou triturer.

4. Forme pharmaceutique intraorale à parties multiples selon l'une quelconque des revendications précédentes, comprenant un tampon et/ou un agent d'ajustement du pH qui, après administration à un sujet, élève de façon transitoire le pH de la salive du sujet de 0,2 à 3,5 unités de pH, de préférence de 0,5 à 2,0 unités de pH.

5. Forme pharmaceutique intraorale à parties multiples selon l'une quelconque des revendications 2 à 4, où au moins une des parties à désintégration rapide et lente comprend un composant pour créer une sensation organoleptique notable ; où, de préférence la ou les sensation(s) organoleptique(s) est/sont sous la forme d'un ou plusieurs d'une perception de ou un changement de perception d'un arôme, un refroidissement, une brûlure, un réchauffement, un croustillement, un picotement, la formation de bulles, un moussage, une effervescence, l'hydratation buccale, une forme physique, une adhérence et une texture, par exemple une dureté, une mollesse, une rugosité et des gravages ; où, de préférence, la ou les sensation(s) organoleptique(s) est/sont telle(s) qu'elle(s) permette(nt) à un sujet utilisant la forme pharmaceutique de différencier différentes parties de celle-ci ; et où, de préférence, la sensation organoleptique est une perception d'arôme ou un changement de perception d'arôme.

6. Forme pharmaceutique intraorale à parties multiples selon la revendication 5, où une sensation organoleptique est délivrée depuis une partie en tant que signal pour informer un sujet utilisant la forme pharmaceutique qu'une certaine fraction de, par exemple, plus des trois quarts de, la totalité ou quasiment la totalité de, une substance pharmaceutiquement active étant initialement présente dans ladite partie a été libérée depuis celle-ci.

7. Forme pharmaceutique intraorale à parties multiples selon la revendication 5 ou la revendication 6, où une sensation organoleptique est délivrée depuis une partie en tant que signal pour informer un sujet utilisant la forme pharmaceutique qu'une substance pharmaceutiquement active étant présente dans ladite partie a commencé à être libérée depuis celle-ci.

8. Forme pharmaceutique intraorale à parties multiples selon l'une quelconque des revendications précédentes, où
I. les parties à désintégration rapide et lente comprennent le même agent pharmaceutiquement actif, ou
II. les parties à désintégration rapide et lente comprennent des agents pharmaceutiquement actifs différents, ou
III. les parties à désintégration rapide et lente, indépendamment de leurs temps de désintégration respectifs, comportent des agents pharmaceutiquement actifs identiques ou différents, et/ou
IV. la forme pharmaceutique permet d'inclure des composants non compatibles, tels que des composants d'arôme, des tampons et des agents pharmaceutiquement actifs qui ne sont pas compatibles, par formulation de tels composants dans des parties séparées.

9. Forme pharmaceutique intraorale à parties multiples selon l'une quelconque des revendications précédentes, où le temps de désintégration pour la partie à désintégration plus lente est 3 à 10 fois plus longue, de préférence 3 à 5 fois plus longue, que pour la partie à désintégration la plus rapide.

10. Forme pharmaceutique intraorale à parties multiples selon l'une quelconque des revendications précédentes, où la ou les partie(s) à désintégration rapide comprend/comprennent un effervescent.

11. Forme pharmaceutique intraorale à parties multiples selon l'une quelconque des revendications précédentes, où l'au moins une partie à désintégration rapide recouvre au moins partiellement l'au moins une partie à désintégration lente.

12. Forme pharmaceutique intraorale à parties multiples selon l'une quelconque des revendications précédentes, où la ou les partie(s) à désintégration lente recouvre(nt) au moins partiellement la surface de la partie à désintégration rapide.

13. Forme pharmaceutique intraorale à parties multiples selon l'une quelconque des revendications précédentes, étant une pastille, un comprimé, un film oral, un comprimé sublingual, un trochisque, une sucette, un bonbon dur, une lentille en chocolat, une microbille, une gelée, une dragée tendre, une gomme au vin, un semi-solide, une forme pharmaceutique à remplissage central ou une combinaison de ceux-ci.

14. Forme pharmaceutique intraorale à parties multiples selon l'une quelconque des revendications précédentes, dans laquelle chaque partie de désintégration est composée de deux sous-parties ou plus, chaque sous-partie comprenant un agent pharmaceutiquement actif, où l'agent pharmaceutiquement actif dans au moins une desdites sous-parties est revêtu.

15. Forme pharmaceutique intraorale à parties multiples selon l'une quelconque des revendications précédentes comprenant un composant pour traiter la dépendance au tabac, qui est choisi dans le groupe constitué de : un sel de nicotine, la forme de base libre de nicotine, un dérivé de nicotine, tel qu'un échangeur de cation de nicotine, un complexe d'inclusion de nicotine ou la nicotine dans une liaison non covalente quelconque, la nicotine liée à des zéolites, la nicotine liée à la cellulose ou des microsphères d'amidon, un promédicament de nicotine, et/ou des mélanges de ceux-ci ; où le complexe d'inclusion de nicotine est de préférence un complexe de cyclodextrine, où la cyclodextrine utilisée est choisie parmi la α-, β- et γ-cyclodextrine, des dérivés hydroxypropyle de α-, β- et γ-cyclodextrine, des sulfoalkyléther-cyclodextrines tels que le sulfobutyléther-β-cyclodextrine, des cyclodextrines alkylées telles que la β-cyclodextrine méthylée de façon aléatoire, et des cyclodextrines ramifiées telles que la glucosyl- et maltosyl-β-cyclodextrine ; et où l'échangeur de cation de nicotine est de préférence un échangeur de cation de polyacrylate ; et où le sel de nicotine est, de préférence, un sel de monotartrate, hydrogénotartrate, citrate, malate et/ou chlorhydrate ; où ladite nicotine et/ou les métabolites de celle-ci est présente en une quantité de 0,05 à 12 mg, de préférence en une quantité de 0,1 à 6 mg, plus préférablement en une quantité de 1 à 6 mg, et de manière préférée entre toutes en une quantité de 2 à 5 mg calculée en forme de base libre de nicotine par dose unitaire.

16. Forme pharmaceutique intraorale à parties multiples selon l'une quelconque des revendications précédentes, comprenant un composant pour traiter la dépendance au tabac étant choisi parmi l'un ou plusieurs de la varénicline, le bupropion, la nortriptyline, la doxépine, la fluoxétine, l'imipramine, le moclobémide, et/ou la cytisine.

17. Forme pharmaceutique intraorale à parties multiples selon l'une quelconque des revendications 1 à 16 pour utilisation dans l'obtention d'une réduction rapide et/ou prolongée et/ou totale de l'envie de fumer ou de la consommation de tabac.

18. Forme pharmaceutique intraorale à parties multiples selon l'une quelconque des revendications 1 à 16, dans laquelle au moins une partie à désintégration rapide comprend un excipient compressible choisi parmi l'isomalt, le dextrose monohydraté, la maltodextrine, le lactose monohydraté, la dextrine, le mannitol, le lactitol, le sorbitol, le xylitol, l'érythritol, le saccharose et le lactose, et des mélanges ou dérivés de ceux-ci ; où, de préférence, la partie à désintégration rapide comprend au moins 40 % en poids d'un excipient compressible choisi parmi l'isomalt, le dextrose monohydraté, la maltodextrine, le lactose monohydraté, la dextrine, le mannitol, le lactitol, le sorbitol, le xylitol, l'érythritol, le saccharose et le lactose, et des mélanges de ceux-ci ; où, de préférence, l'excipient compressible est sous la forme de particules avec un diamètre de particule moyen d'environ 50 à environ 400 microns.

19. Forme pharmaceutique intraorale à parties multiples selon la revendication 18, dans laquelle le rapport en poids de l'excipient compressible à l'excipient gonflable dans l'eau est d'environ 10:1 à environ 500:1.

20. Forme pharmaceutique intraorale à parties multiples selon l'une quelconque des revendications 1 à 16, dans laquelle au moins une partie à désintégration rapide comprend en outre un composant tampon de pH et/ou un composant d'ajustement de pH choisi parmi un carbonate comprenant le bicarbonate ou le sesquicarbonate, le glycinate, le phosphate, le glycérophosphate ou le citrate d'un métal alcalin, tel que le potassium ou le sodium, ou l'ammonium, comprenant le phosphate trisodique, l'hydrogénophosphate disodique ; le phosphate tripotassique, l'hydrogénophosphate dipotassique, et l'hydroxyde de calcium, le glycinate de sodium, le trométamol ou un acide aminé ; et des mélanges de ceux-ci.

21. Forme pharmaceutique intraorale à parties multiples selon l'une quelconque des revendications 1 à 16 ou 18 à 20, dans laquelle la partie ou les parties à désintégration rapide a/ont une dureté inférieure à environ 15 kp/cm², et la partie ou les parties à désintégration lente a/ont une dureté supérieure à environ 15 kp/cm².

22. Forme pharmaceutique intraorale à parties multiples selon l'une quelconque des revendications 1 à 16 ou 18 à 21, dans laquelle la partie ou les parties à désintégration lente comprend/comprennent un excipient choisi dans, mais non limité à, le groupe constitué de : l'isomalt, le saccharose, le dextrose, le dextrose monohydraté, le sirop de maïs, le lactitol, la lycasine, le mannitol, le sorbitol, l'érythritol, le xylitol, des amidons, des amidons gélatinisés, la maltodextrine, le lactose, le lactose monohydraté, la dextrine et des mélanges et/ou dérivés de ceux-ci.

23. Forme pharmaceutique intraorale à parties multiples selon l'une quelconque des revendications 1 à 16 ou 18 à 22, dans laquelle la partie ou les parties à désintégration lente comprend/comprennent au moins 50 %, en poids, d'un sucre choisi parmi l'isomalt, le saccharose, le dextrose, le sirop de maïs, le lactitol, et la lycasine, et des mélanges et/ou dérivés de ceux-ci ; où, de préférence, la partie ou les parties à désintégration rapide comprend/comprennent en outre un couple effervescent comprenant un membre choisi dans le groupe constitué des bicarbonate de sodium, bicarbonate de potassium, carbonate de calcium, carbonate de magnésium, et carbonate de sodium et un membre choisi dans le groupe constitué de l'acide citrique, l'acide malique, l'acide fumarique, l'acide tartrique et l'acide alginique.

24. Forme pharmaceutique intraorale à parties multiples selon l'une quelconque des revendications 1 à 16 ou 18 à 23, dans laquelle l'agent pharmaceutiquement actif est sous la forme de particules qui sont en outre revêtues avec un polymère de masquage de goût et dans laquelle le diamètre de particule moyen des particules est d'environ 50 microns à environ 1000 microns.

25. Forme pharmaceutique intraorale à parties multiples selon l'une quelconque des revendications 1 à 16 ou 18 à 24, dans laquelle au moins une des parties à désintégration lente comprend une pluralité d'ouvertures exposant la surface de cette/ces partie(s), et recouvre sensiblement la surface d'au moins une des parties à désintégration rapide, où ladite/lesdites partie(s) à désintégration lente comprend/comprennent en outre une pluralité de renfoncements qui, lors d'un contact avec les fluides dans la cavité buccale, sont adaptés pour dissoudre et exposer la surface de la ou les partie(s) à désintégration rapide.

26. Forme pharmaceutique intraorale à parties multiples selon l'une quelconque des revendications 1 à 16 ou 18 à 25, dans laquelle un agent pharmaceutiquement actif dans une partie à désintégration rapide est choisi dans le groupe constitué des zinc, chlorhexidine, L. reuteri, nystatine, amphotéricine, miconazole, phényléphrine, dextrométhorphan, pseudoéphédrine, acétaminophène, ibuprofène, kétoprofène, lopéramide, famotidine, carbonate de calcium, siméthicone, pseudoéphédrine, chlorphéniramine, méthocarbomal, chlophédianol, acide ascorbique, menthol, pectine, dyclonine, benzocaïne, et menthol, et des sels pharmaceutiquement acceptables et dérivés de ceux-ci.

27. Forme pharmaceutique intraorale à parties multiples selon l'une quelconque des revendications 1 à 16 ou 18 à 26, dans laquelle la face d'au moins une partie a une forme convexe et la face d'une partie adjacente a une forme concave.

28. Forme pharmaceutique intraorale à parties multiples selon l'une quelconque des revendications 1 à 16 ou 18 à 27 ayant des similarités géométriques à une sphère, un objet oblongue ouvert ou fermé, un sandwich, un hamburger ou un tore.

29. Forme pharmaceutique intraorale à parties multiples selon l'une quelconque des revendications 1 à 16 ou 18 à 28 ayant une ou des couches(s) inter-parties comprenant un matériau de type adhésif comestible ; où, de préférence le matériau de type adhésif comestible comprend un composant choisi dans le groupe constitué des polyéthylène glycol, poly(oxyde d'éthylène), polycaprolactone, cire de carnauba, cire microcristalline, oppanol, cire de laque et cire d'abeilles.

30. Forme pharmaceutique intraorale à parties multiples selon l'une quelconque des revendications 1 à 16 ou 18 à 29, dans laquelle au moins une partie à désintégration rapide comprend au moins un agent pharmaceutiquement actif choisi dans le groupe de la phényléphrine, le dextrométhorphan, la chlorphéniramine, le chlophédianol, et la pseudoéphédrine, et dans laquelle au moins une partie à désintégration lente comprend au moins un agent pharmaceutiquement actif choisi dans le groupe des menthol, nicotine, dyclonine, pectine, benzocaïne, thymol, salicylate de méthyle et eucalyptol.

31. Forme pharmaceutique intraorale à parties multiples selon l'une quelconque des revendications 1 à 16 ou 18 à 30 où au moins une partie à désintégration rapide est sensiblement exempte de nicotine, où sensiblement exempt est défini comme contenant 0,05 mg par dose unitaire ou moins.

32. Forme pharmaceutique intraorale à parties multiples selon l'une quelconque des revendications 1 à 16 ou 18 à 31 où au moins une partie à désintégration rapide est une partie comprimée et où au moins une partie à désintégration lente comporte une matrice qui est un glaçage de bonbon dur.

33. Forme pharmaceutique intraorale à parties multiples selon l'une quelconque des revendications précédentes, où la ou les partie(s) à désintégration lente ou rapide ont des renfoncement (s) et/ou des trous remplis par la ou les autre(s) partie(s) de désintégration.

34. Forme pharmaceutique intraorale à parties multiples selon l'une quelconque des revendications 1 à 16 ou 18 à 33 comprenant la nicotine et/ou des métabolites de celle-ci, tels que la cotinine, le N'-oxyde de nicotine, la nornicotine, le (S)-nicotine-N-β-glucuronide et des mélanges, isomères, sels et complexes de ceux-ci sous une forme quelconque pour utilisation en thérapie où la thérapie est le traitement d'une maladie choisie dans le groupe constitué de la dépendance au tabac ou à la nicotine, la maladie d'Alzheimer, la maladie de Crohn, la maladie de Parkinson, le syndrome de Tourette, la colite ulcéreuse.

35. Forme pharmaceutique intraorale à parties multiples selon l'une quelconque des revendications précédentes, **caractérisée en ce que** les un ou plusieurs agent(s) pharmaceutiquement actif(s) est/sont choisis parmi les agents anti-inflammatoires diclofénac, kétorolac, indométacine, tomoxicam, piroxicam, ténoxicam, kétoprofène, célécoxib et roficoxib ; les myorelaxants orphénadrine et baclofène ; les médicaments affectant la minéralisation osseuse acide alendronique et acide risédronique ; les analgésiques propoxyphène, buprénorfine, kétobénidon, hydromorphone, tramadol, morphine et tapentadol ; des préparations antimigraineuses ; la dihydroergotamine, l'ergotamine, l'élétriptan, le naratriptan, le rizatriptan, le sumatriptan et le zolmitriptan ; les médicaments antiparkinsoniens pramipexole, ropinirole et sélégiline ; les anxiolytiques alprazolam, diazépam, lorazépam et oxazépam ; les hypnotiques flunitrazépam, midazolam, nitrazépam, triazolam, zaléplone, zopiclone, zolpidem, clométiazole et propiomazine ; le psychostimulant caféine ; les médicaments contre la dépendance à une substance bupropione, lobéline, naltrexone et méthadone ; le remède contre les ulcères gastriques famotidine ; l'antispasmodique hyoscyamine ; les antiémétiques métoclopramide, ondansétron, scopolamine, hyoscine, perfénazine, proclopérazine et halopéridol ; l'agent antidiabétique rosiglitazone ; les agents cardiovasculaires étiléfrine, trinitrate de glycéryle, dinitrate d'isosorbide et mononitrate d'isosorbide ; l'agent antihypertenseur hydralazine ; les diurétiques furosémide et amiloride ; les agents bêta-bloquants propranolol et timolol ; l'inhibiteur calcique amlodipine ; les inhibiteurs d'ACE kaptopril, lisinopril et fosinopril ; l'agent réducteur de lipide sérique simvastatine ; l'antipsoriasique acitrétine ; l'antiasthmatique terbutaline ; les antitussifs codéine et noscapine, et les antihistaminiques clémastine, chlorphéniramine, cyproheptadine, loratadine et acrivastine ; le médicament antidépresseur et contre les troubles sexuels dapoxétine ; les médicaments contre les troubles sexuels sildénafil (Viagra), tadalafil, vardénafil, cabergoline et pramipexole, l'antiépileptique topiramate, et
l'agent favorisant la santé buccale et/ou gastro-intestinale et/ou générale Lactobacillus reuteri.

36. Forme pharmaceutique intraorale à parties multiples selon la revendication 35, **caractérisée en ce qu'**elle comprend de la nicotine et Lactobacillus reuteri.

37. Forme pharmaceutique intraorale à parties multiples selon la revendication 35, **caractérisée en ce qu'**elle comprend de la terbutaline et de la loratadine.
